# EUROPÄISCHE PATENTSCHRIFT

(11) **EP 2 785 334 B1**
(45) Veröffentlichungstag und Bekanntmachung des Hinweises auf die Patenterteilung: **25.09.2024**
(21) Anmeldenummer: 12791163.4
(22) Anmeldetag: 22.11.2012
(51) Int. Cl.: A61K 9/70, A61K 31/4468, A61P 25/00, A61P 25/04

(54) **TRANSDERMALES THERAPEUTISCHES SYSTEM ZUR VERABREICHUNG VON FENTANYL ODER EINEM ANALOGSTOFF DAVON**
TRANSDERMAL THERAPEUTIC SYSTEM FOR APPLICATION OF FENTANYL OR AN ANALOGUE MATERIAL THEREOF
SYSTÈME THÉRAPEUTIQUE TRANSDERMIQUE DESTINÉ À L'APPLICATION DE FENTANYL OU D'UNE MATIÈRE ANALOGUE

(30) Priorität: 30.11.2011 EP 11191254
(43) Veröffentlichungstag der Anmeldung: 08.10.2014
(73) Patentinhaber: Luye Pharma AG, 83714 Miesbach (DE)
(72) Erfinder: SCHURAD, Björn, 81667 München (DE); SCHMITT, Sonja, 82008 Unterhaching (DE)
(74) Vertreter: Kraus & Lederer PartGmbB
(86) Internationale Anmeldenummer: PCT/EP2012/073314
(87) Internationale Veröffentlichungsnummer: WO 2013/079386

(56) Entgegenhaltungen:
- EP-A1- 1 552 822
- EP-A1- 1 552 835
- EP-A1- 2 042 174
- EP-A1- 2 295 046
- US-A- 4 588 580
- US-A1- 2008 175 890

## Beschreibung

Gegenstand der vorliegenden Anmeldung ist ein System zur transdermalen Verabreichung von Fentanyl oder einem Analogstoff davon für therapeutische Zwecke. Das transdermale therapeutische System (TDS, Wirkstoffpflaster) zeichnet sich durch eine hervorragende Kombination der für ein solches System relevanten Eigenschaften aus, insbesondere ausreichende Klebekraft und Hautverträglichkeit über einen langen Zeitraum, so dass eine Tragedauer des Systems von mindestens 3 Tagen gewährleistet ist, die Abwesenheit von kaltem Fluss, geringe Wirkstoffbeladung und ausgezeichnete Wirkstofffreisetzung.

Fentanyl und dessen Analogstoffe, insbesondere Alfentanil, Carfentanil, Lofentanil, Remifentanil, Sufentanil, Trefentanil und ähnliche Verbindungen sind wirksame synthetische Opiate. Fentanyl und dessen Analogstoffe sind hochwirksam und werden schnell metabolisiert. Problematisch bei diesen Verbindungen ist, dass sie einen relativ engen therapeutischen Index besitzen. Bei Überschreiten der Grenzwerte treten unerwünschte Nebeneffekte auf, insbesondere eine Beeinträchtigung der Atmung, die - wenn nicht geeignete Gegenmaßnahmen ergriffen werden - zum Tod führen kann. Die Wirkstoffe sind relativ teuer und es besteht ein sehr hohes Missbrauchsrisiko. Deshalb müssen Fentanylpflaster einerseits eine sehr präzise gesteuerte Freisetzung des Wirkstoffs gewährleisten und andererseits soll das Produkt so geschaffen sein, dass der Wirkstoff hieraus nicht leicht zu Missbrauchszwecken entfernt werden kann. Die Pflaster sind in der Regel für einen Einsatz von mindestens drei Tagen vorgesehen und müssen über diesen Zeitraum ausreichend auf der Haut kleben.

Ein Wirkstoffpflaster ist typischerweise eine kleine klebende Bandage, welche den abzugebenden Wirkstoff enthält. Diese Bandagen können verschiedene Formen und Größen haben. Der einfachste Typ ist ein Klebe-Monolith, welcher einen Wirkstoffvorrat (Reservoir) auf einem Träger umfasst. Das Reservoir wird typischerweise aus dem Wirkstoff in einem pharmazeutisch akzeptablen druckempfindlichen Klebstoff gebildet. Es kann aber auch aus einem nicht (oder schlecht) klebenden Material geformt sein, dessen Haut-Kontaktfläche mit einer dünnen Schicht eines geeigneten Klebstoffes versehen ist.

Komplexere Pflaster sind Mehrfachlaminate oder Pflaster mit Wirkstoffvorrat (der gegebenenfalls in einer Flüssigkeit gelöst vorliegen kann), in welchen zwischen dem Wirkstoffvorrat und dem die Haut kontaktierenden Klebstoff eine die Wirkstoff-Freisetzung steuernde Membran angeordnet sein kann. Diese Membran dient dazu, die Auswirkungen von Variationen der Hautdurchlässigkeit durch Herabsetzung der in vitro- und in vivo-Abgaberate des Wirkstoffs aus dem Pflaster zu kontrollieren und gegebenenfalls zu vermindern.

Ein Wirkstoffpflaster kann den Wirkstoff entweder vollständig gelöst im Vorrat enthalten oder der Vorrat kann einen Überschuss ungelösten Wirkstoffs enthalten (Depot-Pflaster). Die Anwesenheit von ungelöstem Wirkstoff oder anderen Bestandteilen in einem Pflaster kann bei der Lagerung sowie beim Gebrauch allerdings Stabilitäts- und andere Probleme aufwerfen. Eine Schwierigkeit besteht auch darin, dass sichergestellt werden muss, dass sich der Wirkstoff aus dem festen Depot ausreichend schnell nachlöst, um den abgegebenen Wirkstoff zu ersetzen. Wirkstoffpflaster, deren Reservoir feste Wirkstoffteilchen aufweist, werden im Stand der Technik daher häufig als nachteilig angesehen.

Aus dem Stand der Technik sind viele verschiedene transdermale Pflaster für die Verabreichung von Fentanyl bekannt. Die WO 02/074286 beschreibt ein transdermales Pflaster mit einem Reservoir enthaltend Fentanyl, wobei das Reservoir eine polymere Zusammensetzung, bevorzugt Polyacrylat, in einheitlichem Phasenzustand aufweist, die frei ist von nicht gelöstem Wirkstoff. Eine Übersättigung soll hier ausdrücklich vermieden werden.

Es gibt viele Versuche, Fentanylpflaster auch auf der Basis einer Matrixschicht aus Polyisobutylen herzustellen. Erste derartige Versuche werden bereits in dem Grundpatent zu Fentanylpflastern, dem US-A 4,588,580, beschrieben. Diese Druckschrift offenbart ein transdermales therapeutisches System mit einer Polyisobutylenmatrix und Mineralöl, die eine 2%ige Beladung von Fentanyl als ungelöstem Feststoff enthält. Das System weist in der Praxis jedoch Nachteile auf, und in der Folgezeit ging die Entwicklung daher von Polyisobutylenmatrices weg bzw. falls Polyisobutylenmatrices verwendet wurden, versuchte man, den Wirkstoff vollständig in der Polyisobutylenmatrix zu lösen.

Ein transdermales therapeutisches System mit einer Polyisobutylenmatrix ist in Roy et al., Journal of Pharmaceutical Sciences, Vol. 85, Nr. 5, Mai 1996, Seiten 491 bis 495 beschrieben. Es wird gezeigt, dass bei Konzentrationen von Fentanyl in der Polyisobutylenmatrix von mehr als 4% Wirkstoff ausfällt, und dies wurde offensichtlich von Roy et al. als negativ angesehen. Roy et al. schlägt für Pflaster mit geringer Wirkstoffbeladung an Fentanyl Polysilikonpflaster vor und keine Polyisobutylenpflaster.

In der US 2007/0009588 und der US 2006/0013865 werden dementsprechend Polyisobutylenmatrices vorgeschlagen, bei denen sorgfältig darauf zu achten ist, dass der Wirkstoff in der Polyisobutylenmatrix vollständig gelöst vorliegt. Das Auftreten von Kristallen in der Matrix wird als negativ angesehen. Die Polyisobutylenmatrices enthalten Polyisobutylene unterschiedlichen Molekulargewichts und ein Mineralöl, bei dem es sich bevorzugt um flüssiges Paraffin handelt, oder ein Styrol-Isopren-Styrol Blockpolymer.

Die DE 198 37 902 offenbart transdermale therapeutische Systeme auf der Basis von Polyisobutylen, die insbesondere zur Verabreichung von Clonidin geeignet sind, unter den dort genannten Wirkstoffen findet sich aber auch Fentanyl. Beispiele für Fentanylpflaster finden sich in dieser Druckschrift nicht, ein Hinweis darauf, dass bei den dort offenbarten Pflastern der Wirkstoff als Feststoff vorhanden sein soll, findet sich in der Druckschrift ebenfalls nicht. Die Druckschrift enthält keine in vivo-Untersuchungen zur Freisetzung des Wirkstoffs aus den Pflastern. Die Polyisobutylenschicht dieser Pflaster enthält mindestens 5 Gew.-% eines Füllstoffs.

In der WO 2009/130039 werden transdermale therapeutische Systeme zur Verabreichung von Fentanyl oder einem Analogstoff davon beschrieben, bei denen auf einer Rückschicht eine Polyisobutylenschicht aufgebracht ist, die den Wirkstoff enthält und die einen Gehalt an Gelbildner von höchstens 4 Gew.-% aufweist. Mit diesen Pflastern soll eine längere und gleichmäßigere Freisetzung erzielt werden, als mit den bekannten Fentanylpflastern.

Die EP 0 272 987 offenbart ebenfalls ein Pflaster zur Verabreichung von Wirkstoffen, wobei eine besonders ausgestaltete Trägerschicht als relevant angesehen wird. In einem Beispiel der Druckschrift wird ein Fentanylpflaster offenbart, das zwei Wirkstoffschichten enthält, wobei die eine Wirkstoffschicht eine Polyisobutylenschicht, die andere eine Polydimethylsiloxanschicht darstellt. Die Druckschrift offenbart, dass der Wirkstofffluss aus der Polyisobutylenschicht wesentlich niedriger ist, als aus der Polydimethylsiloxanschicht.

Die WO 2011/029948 offenbart ein transdermales therapeutisches System zur Verabreichung von Fentanyl oder einem Analogstoff des Fentanyls durch die Haut bei der die Matrixschicht aus zwei Schichten besteht, die jeweils ein Polyisobutylen mit einer anderen Zusammensetzung enthalten. Der Aufbau der Pflaster ist durch die Mehrfachbeschichtung relativ komplex.

Die US 2011/0020426 offenbart ein Verfahren zur Herstellung einer Klebstoffzusammensetzung aus einem Polyisobutylen mit hohem Molekulargewicht und einem Polyisobutylen mit geringerem Molekulargewicht. Diese Klebstoffzusammensetzung ist für Pflaster zur Verabreichung von Wirkstoff geeignet. Die Wirkstoffe sind nicht besonders eingeschränkt und Fentanyl wird als ein Wirkstoff aus einer langen Liste von Wirkstoffen offenbart. Keines der Beispiele betrifft ein Fentanylpflaster.

Die EP 1 625 845 offenbart ein transdermales therapeutisches System für die Verabreichung von Fentanyl aus einer Klebstoffschicht aus zwei Arten von Polyisobutylen mit unterschiedlichem Molekulargewicht. Die Klebstoffschicht enthält weiterhin noch einen Klebrigmacher und eine organische Flüssigkeit, bei der es sich um Polybuten handeln kann. Wie in der US 2007/0009588 und der US 2006/0013865 sieht es die EP 1 625 845 als wesentlich an, dass das Fentanyl in der Klebstoffschicht vollständig gelöst ist. Dementsprechend werden der Klebrigmacher und die organische Flüssigkeit ausgewählt, um eine vollständige Lösung des Fentanyls in der Klebstoffschicht zu gewährleisten. Bei der organischen Flüssigkeit handelt es sich bevorzugt um ein Gemisch aus einem Fettsäurealkylester und einem verzweigten langkettigen Alkohol, da in diesem Gemisch die Löslichkeit des Fentanyls am Stärksten ist.

Es ist eine ausgesprochen anspruchsvolle Aufgabe, ein transdermales therapeutisches System für die Verabreichung von Fentanyl oder einem Analogstoff davon zur Verfügung zu stellen, das alle praxisrelevanten Eigenschaften vorteilhaft verbindet. Ein derartiges transdermales therapeutisches System muss nicht nur über die gesamte Anwendungsdauer eine ausreichende Wirkstofffreisetzung gewährleisten, sondern darüber hinaus auch eine ausreichende Klebekraft und Hautverträglichkeit damit die für Fentanyl und Analogstoffe davon übliche Anwendungsdauer von typischerweise drei Tagen gewährleistet ist. Ferner darf bei dem transdermalen therapeutischen System kein nennenswerter kalter Fluss auftreten. Bei einem solchen kalten Fluss wird das transdermale therapeutische System während der Lagerung praktisch unbrauchbar. Schließlich sollte das Pflaster auch noch einfach und kostengünstig herzustellen sein, mit möglichst wenig Wirkstoffbeladung auskommen und einen möglichst geringen Restwirkstoffgehalt nach Gebrauch aufweisen.

Die im Stand der Technik vorgeschlagenen Pflaster auf Polyisobutylenbasis erfüllen Teilaspekte der vorstehenden erwünschten Eigenschaften. Beispielsweise zeigt das in der EP 1 625 845 beschriebene Pflaster mit einer Matrixschicht aus zwei Polyisobutylenen mit unterschiedlichem Molekulargewicht eine zufriedenstellende Freisetzung des Wirkstoffs, allerdings ist die Klebleistung der Pflaster nicht zufriedenstellend. Bei nicht hinreichender Klebeleistung des Pflasters ist selbstverständlich eine Anwendung über einen längeren Zeitraum, z.B. von 3 Tagen oder mehr, nicht zuverlässig möglich. Auch ist die Menge an Wirkstoff, die in das Pflaster eingebracht wird, für ein Mehrtagespflaster zur Behandlung von starken Schmerzen zu gering. In der WO 2011/029948 wurde ein Weg gefunden, die Klebeeigenschaften der Fentanylpflaster auf Polyisobutylenbasis zu verbessern und gleichzeitig eine ausgezeichnete Wirkstofffreisetzung zu erzielen. In die Pflaster kann eine Menge an Wirkstoff eingebracht werden, die auch zur Behandlung starker Schmerzen über mehrere Tage ausreichend ist. Allerdings neigen die dort offenbarten Pflaster dazu, kalten Fluss zu zeigen und der Aufbau der Pflaster mit zwei übereinander angeordneten haftklebenden Schichten aus verschiedenen Polyisobutylenen ist relativ komplex was die Herstellung des Pflasters aufwendig und teuer macht.

Desweiteren zeigen die bekannten Pflaster einen signifikanten Restwirkstoffgehalt nach Gebrauch. Gemäß H.G. Kress et al., European Journal of Pharmaceutics and Biopharmaceutics, 75(2010), 225-231 ist der gemessene Restfentanylgehalt in kommerziell erhätlichen Matrifen^{®} und Durogesic DTrans^{®} Pflastern im Mittel 82.3% bzw. 52.3%, obwohl ihre Freisetzung bioequivalent ist. Entsprechend ist der Gesamtgehalt in Durogesic DTrans^{®} vor Gebrauch deutlich höher. Ein Restgehalt an Wirkstoff nach Gebrauch im Bereich von 50-80% ist jedoch nicht nur aus Wirtschaftlichkeitsaspekten nachteilig, sondern stellt insbesondere ein signifikantes Missbrauchsrisiko dar.

Es besteht daher weiterhin Bedarf nach einem Pflaster auf der Basis von Polyisobutylenen zur Verabreichung von Fentanyl oder einem Analogstoff davon, das die vorstehend genannte vorteilhafte Kombination an Eigenschaften aufweist, das also nicht nur eine hervorragende Freisetzung des Wirkstoffs sondern auch sehr gute Klebeeigenschaften und hervorragenden Tragekomfort (jeweils von drei Tagen oder mehr), zur Verfügung stellt, dabei bei der Lagerung keinen kalten Fluss zeigt und das leicht und kostengünstig mit geringem Wirkstoffeinsatz und geringem Restwirkstoffgehalt nach Gebrauch hergestellt werden kann.

Die Erfinder der vorliegenden Anmeldung haben nunmehr überraschend gefunden, dass die Temperaturabhängigkeit des viskoelastischen Verhaltens der Polyisobutylene der Matrixschicht eines transdermalen therapeutischen Systems von entscheidender Bedeutung für die relevanten Eigenschaften des transdermalen therapeutischen Systems ist, insbesondere für die Kombination der Eigenschaften a) gute Klebeeigenschaft, b) Minimierung von kaltem Fluss, c) hohe Wirkstofffreisetzung über mehrere Tage und d) geringem Restwirkstoffgehalt nach Gebrauch. Es wurde gefunden, dass transdermale therapeutische Systeme, bei denen die Matrixschicht aus Polyisobutylenen aufgebaut ist, deren viskoelastische Eigenschaften eine bestimmte Temperaturabhängigkeit zeigen, die Kombination der vorstehend genannten hervorragenden Eigenschaften aufweisen, insbesondere wenn die Matrixschicht noch einen Permeationsverstärker und einen Klebrigmacher enthält. Überraschenderweise wurde gefunden, dass insbesondere die Temperaturabhängigkeit des Speichermoduls der in der Matrixschicht vorhandenen Polyisobutylene eine entscheidende Rolle spielt, um kalten Fluss zu vermeiden, gleichzeitig eine hohe Klebkraft bei sehr guter Hautverträglichkeit über einen Zeitraum von mindestens drei Tagen zur Verfügung zu stellen und trotzdem eine ausreichende Freisetzung des Wirkstoffs und geringem Restwirkstoffgehalt zu gewährleisten. Ein komplexer mehrschichtiger Aufbau, wie er beispielsweise in der WO 2011/029948 verlangt wird, ist bei den erfindungsgemäßen transdermalen therapeutischen Systemen nicht erforderlich.

Die vorliegende Anmeldung betrifft damit ein transdermales therapeutisches System zur Linderung von Schmerz durch Verabreichung eines Wirkstoffs durch die Haut während einer vorgesehenen Tragezeit von 3 bis 7 Tagen bestehend aus
a) einer Rückschicht,
b) einer haftklebende Matrixschicht, die den Wirkstoff enthält, und
c) einer Abziehschicht (Release-Liner),
sowie gegebenenfalls
b1) einer zusätzlichen Klebstoffschicht oder einem Overtape, die die Haftung des transdermalen therapeutischen Systems auf der Haut verbessert und die auf der haftklebenden Matrixschicht aufgebracht sind, oder
b2) einer Membran, die die Freisetzung des Wirkstoffes kontrolliert und auf der sich eine Klebstoffschicht befindet, wobei die Membran auf der haftklebenden Matrixschicht aufgebracht ist,
wobei der Wirkstoff Fentanyl oder ein Analogstoff des Fentanyls ausgewählt aus Alfentanil, Lorfentanil, Lofentanil, Remifentanil und Trefentanil oder ein Salz eines dieser Wirkstoffe ist und
wobei die Matrixschicht frei von Gelbildnern ist und
als haftklebendes Polymer ein Gemisch aus einem Polyisobutylen A und einem Polyisobutylen B enthält,
wobei der Gehalt des Polyisobutylens A zum Polyisobutylen B in der Matrixschicht im Bereich von 20% (A) : 80% (B) bis 40% (A) : 60% (B) liegt, jeweils bezogen auf das Gesamtgewicht der Polyisobutylene A und B, und
wobei das Polyisobutylen A ein Speichermodul G' aufweist, dessen Wert im Temperaturbereich von 10°C bis 40°C im Wesentlichen konstant ist und
wobei das Polyisobutylen B ein Speichermodul G' aufweist, dessen Wert im Temperaturbereich von 10°C bis 40°C mit steigender Temperatur kontinuierlich abnimmt,
wobei das Speichermodul G' im linearen viskoelastischen Bereich bei einer Frequenz von 10 rad/sec unter Verwendung eines Rheometers mit paralleler Plattengeometrie und parallelen Platten gemessen wird und
wobei die haftklebende Matrixschicht ungelösten Wirkstoff in Form von Wirkstoffpartikeln enthält, und nach Applikation auf der Haut für die Dauer der vorgesehenen Tragezeit, einen Restwirkstoffgehalt von unter 35%, vorzugsweise von 25% des Ausgangsgehalts an Wirkstoff aufweist.

Das erfindungsgemäße transdermale therapeutische System enthält als Wirkstoff Fentanyl oder einen Analogstoff des Fentanyls ausgewählt aus Alfentanil, Carfentanil, Lofentanil, Remifentanil und Trefentanil oder ein Salz einer dieser Wirkstoffe. Am meisten bevorzugt ist der Wirkstoff Fentanyl. Die Erfindung wird im Folgenden im Wesentlichen anhand des Wirkstoffs Fentanyl erläutert. Die Ausführungen gelten aber entsprechend auch für die angegebenen Analogstoffe des Fentanyls.

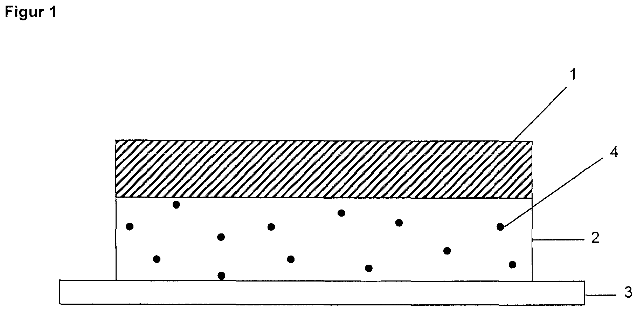

Der bevorzugte Aufbau des erfindungsgemäßen transdermalen therapeutischen Systems in seiner einfachsten Ausgestaltung ist in Figur 1 gezeigt. Auf dem bei der Anwendung der Haut gegenüber befindlichen Ende des transdermalen therapeutischen Systems befindet sich die Rückschicht (1). An der der Haut beim Gebrauch zugewandten Seite der Rückschicht (1) befindet sich die haftklebende Matrixschicht (2), die auch als Reservoir bezeichnet wird. Bei dem erfindungsgemäßen transdermalen therapeutischen System handelt es sich um ein Suspensionspflaster, d.h. der Wirkstoff ist teilweise ungelöst in der haftklebenden Matrixschicht suspendiert. In Figur 1 sind die Wirkstoffteilchen (4) gezeigt.

Wesentlich für die erfindungsgemäßen transdermalen therapeutischen Systeme ist, dass die haftklebende Matrixschicht so viel Wirkstoff enthält, dass ein Teil des Wirkstoffs in ungelöster Form, also als Wirkstoffpartikel, vorliegt.

Bei der Herstellung des erfindungsgemäßen transdermalen therapeutischen Systems wird der Wirkstoff bevorzugt in mikronisierter Form, bei der mehr als 90% der Partikel kleiner als 50 µm, bevorzugt kleiner 25 µm, eingesetzt. Auch in der Matrixschicht des transdermalen therapeutischen Systems liegt der Wirkstoff in mikronisierter Form vor, allerdings können sich durch Umlagerungsreaktionen bei der Herstellung und Lagerung des transdermalen therapeutischen Systems Abweichungen der Teilchengröße ergeben. Auch innerhalb des transdermalen therapeutischen Systems sind vorzugsweise mehr als 90% der Wirkstoffpartikel kleiner als 100 µm, stärker bevorzugt kleiner als 50 µm und insbesondere kleiner als 25 µm. Zur Herstellung des erfindungsgemäßen transdermalen therapeutischen Systems wird bevorzugt mikronisiertes Fentanyl mit einer mittleren Teilchengröße von 1 µm oder mehr, stärker bevorzugt 2 µm oder mehr, eingesetzt. Bei dem eingesetzten Fentanyl sind bevorzugt mehr als 90% der Partikel kleiner 25 µm. Diese Teilchengrößen finden sich bevorzugt ebenfalls in dem fertig hergestellten transdermalen therapeutischen System. In der Matrixschicht des transdermalen therapeutischen Systems kann die Teilchengröße und die Teilchengrößenverteilung der Wirkstoffteilchen am besten durch übliche Lichtmikroskopie bestimmt werden. Die Auswertung erfolgt über übliche Computerprogramme (Bildverarbeitungssysteme), die in der Regel auf die verwendeten Mikroskope abgestimmt sind. Die Teilchengröße bezieht sich auf den Teilchendurchmesser, sofern nichts anderes angegeben oder offensichtlich ist.

Als Ausgangsmaterial für das mikronisierte Fentanyl wird handelsübliches Fentanyl eingesetzt, das an sich für die klinische Anwendung geeignet ist. Derartiges Fentanyl hat üblicherweise eine Verteilung der Partikelgröße dergestalt, dass 90% der Partikel kleiner sind als 2500 µm. Vorzugsweise sind etwa 90% der Partikel kleiner als etwa 1000 µm und/oder mehr bevorzugt 50% der Partikel kleiner als etwa 100 µm.

Erfindungsgemäß kann jedes bekannte Mikronisierungsverfahren verwendet werden, das die gewünschte Teilchengröße zur Verfügung stellt. Bevorzugt wird Fentanyl eingesetzt, das mittels einer üblichen "jet mill" mikronisiert wurde, z.B. einer jet mill vom Typ AS der Hosokawa Alpine AG.

Durch das erfindungsgemäß eingesetzte Mikronisierungsverfahren wird die Größe der Fentanylpartikel bevorzugt so eingestellt, dass die mittlere Teilchengröße in den vorstehend angegebenen Bereichen liegt. Bevorzugt ist ebenfalls, dass mehr als 90% der Partikel kleiner sind als 50 µm, mehr bevorzugt kleiner als 25 µm.

Für die Bestimmung der Teilchengröße bzw. der Teilchengrößenverteilung des Wirkstoffs gibt es verschiedene Verfahren, beispielsweise Lichtbeugungsverfahren (Laserdiffraktometrie), wie sie in den Geräten der Firma Malvern Instruments, z.B. dem "Malvern MasterSizer X", verwendet werden, mechanische Siebrüttelverfahren, wie sie die Firma FMC zur Bestimmung der Korngrößenverteilung ihrer AVICEL PH^{®}-Produkte verwendet, oder auch "air jet"-Siebanalysen, die beispielsweise mit einem ALPINA^{®}-"air jet" Modell 200 ausgeführt werden können.

Sofern nicht anders angegeben, werden die (mittleren) Teilchengrößen bzw. Teilchengrößenverteilungen mit dem Verfahren Laserdiffraktometrie bestimmt, beispielweise mit dem Gerät Mastersizer 2000 von Malvern.

Definiert man den Wirkstoff über die Angabe der mittleren Teilchengröße und die Teilchengrößenverteilung, weist der erfindungsgemäß eingesetzte mikronisierte Wirkstoff bevorzugt eine mittlere Teilchengröße von 20 µm oder weniger auf, und es ist bevorzugt, dass der Wirkstoff eine Korngrößenverteilung (Teilchengrößenverteilung) hat, bei der weniger als 10% der Teilchen eine Größe von 30 µm oder darüber haben und weniger als 10% der Teilchen eine Größe von 1 µm oder darunter haben.

Das erfindungsgemäße transdermale therapeutische System weist ebenfalls eine Abziehschicht (3) auf, die die haftklebende Matrixschicht bedeckt und die vor Gebrauch und Anwendung des Pflasters zu entfernen ist.

In der am stärksten bevorzugten Ausführungsform besteht das erfindungsgemäße transdermale therapeutische System ausschließlich aus der Rückschicht (1) und der haftklebenden Matrixschicht (2), die den Wirkstoff enthält und der vor Gebrauch zu entfernenden Abziehschicht (3).

Es ist auch möglich, dass das transdermale therapeutische System auf der haftklebenden Matrixschicht (2) noch eine zusätzliche Klebstoffschicht oder ein Overtape aufweist die die Haftung des erfindungsgemäßen transdermalen therapeutischen Systems auf der Haut eines Patienten verbessern soll. Bei einem Overtape handelt es sich um eine zusätzliche Klebstoffschicht, die größter ist als die wirkstoffhaltige Schicht bzw. das eigentliche TDS und somit die Haftung verbessert. Erfindungsgemäß wurde aber gefunden, dass eine solche zusätzliche Klebstoffschicht bzw. ein Overtape bei dem erfindungsgemäßen transdermalen therapeutischen System nicht erforderlich ist und daher ist erfindungsgemäß ein transdermales therapeutisches System bevorzugt, das eine solche zusätzliche Klebstoffschicht bzw. ein Overtape nicht enthält. Eine derartige Klebstoffschicht kann aus verschiedenen an sich bekannten Haftklebstoffen bestehen, wie z.B. ebenfalls Polyisobutylen, Polysilikon oder Polyacrylat.

Ebenfalls ist es möglich, dass auf der haftklebenden Matrixschicht (2) noch eine Membran aufgebracht ist, die die Freisetzung des Wirkstoffs kontrolliert. Erfindungsgemäß wurde wiederum gefunden, dass bei dem erfindungsgemäßen Aufbau des transdermalen therapeutischen Systems eine solche Membran nicht erforderlich ist, sie kann aber selbstverständlich (beispielsweise zur Erhöhung der Sicherheit) aufgebracht werden. Geeignete Membranen sind im Stand der Technik bekannt und können beispielsweise auf der Basis von Polypropylen oder Polyethylenvinylacetat aufgebaut sein, beispielsweise kann es sich bei der Membran und eine mikroporöse Polypropylenfolie handeln. Geeignete Membranen sind in der WO 2009/130039 auf Seite 5 offenbart, deren Offenbarung insoweit hier durch Bezugnahme aufgenommen wird.

Da bei den erfindungsgemäßen transdermalen therapeutischen Systemen eine derartige Membran aber nicht zwingend erforderlich ist, weisen die erfindungsgemäßen transdermalen therapeutischen Systeme bevorzugt keine solche Membran auf. Falls eine derartige Membran dennoch vorhanden ist, befindet sich auf ihr noch eine Klebstoffschicht damit das transdermale therapeutische System auf der Haut haftet.

In der besonders bevorzugten Ausführungsform der erfindungsgemäßen transdermalen therapeutischen Systeme bestehen daher die transdermalen therapeutischen Systeme ausschließlich aus der Rückschicht (1), der haftklebenden Matrixschicht (2), die den Wirkstoff enthält und der Abziehschicht (3), die vor Gebrauch des transdermalen therapeutischen Systems zu entfernen ist und zusätzliche Klebstoffschichten oder eine die Abgabe des Wirkstoffs steuernde Membran sind nicht vorhanden.

Die Rückschicht (1) des transdermalen therapeutischen Systems ist in einer bevorzugten Ausführungsform okklusiv (also abschließend). Derartige Rückschichten sind im Stand der Technik bekannt und können beispielsweise aus Polyolefinen, insbesondere Polyethylen, aus Polyestern oder Polyurethanen bestehen. Auch Schichten, die mehrere verschiedene Polymere übereinander angeordnet beinhalten, sind vorteilhaft als Rückschichten in den erfindungsgemäßen transdermalen therapeutischen Systemen einsetzbar. Ein geeignetes Material für die Rückschicht ist beispielsweise ein Polyolefin, das von der Firma Mylan Technologies Inc. unter der Bezeichnung Mediflex^{®} 1000 vertrieben wird. Andere geeignete Materialien umfassen Cellophan, Celluloseacetat, Ethylcellulose, mit Weichmachern versehene Vinylacetatvinylchloridcopolymere, Ethylenvinylacetatcopolymere, Polyethylenterephthalat, Nylon, Polyethylen, Polypropylen, Polyvinylidenchlorid, Ethylenmethacrylatcopolymere, Papier, das gegebenenfalls beschichtet sein kann, textile Gewebe und Polyesterfolien wie Polyethylenterephthalatfolien. Besonders bevorzugt sind auch Aluminiumfolien und Polymer-Metall-Kompositmaterialien. Die Dicke der Rückschicht ist wie im Stand der Technik üblich z.B. im Bereich von 10 µm bis 80 µm, und in den Beispielen wurde eine Rückschicht mit einer nominellen Dicke von etwa 10-55 µm eingesetzt.

Auf der Rückschicht des Pflasters kann sich noch eine Abdeckschicht befinden, wie es im Stand der Technik bekannt ist. Die Abdeckschicht liegt bevorzugt lose auf der Rückschicht auf und wird durch elektrostatische Kräfte gehalten. Derartige Abdeckschichten sind beispielsweise in der EP 1 097 090 beschrieben, auf die insoweit vollinhaltlich Bezug genommen wird. Die Abdeckschicht ist zumindest auf der auf der Rückschicht aufliegenden Seite antihaftbeschichtet, z.B. silikonisiert oder fluoriert.

Die auf der haftklebenden Matrixschicht aufliegende Abziehschicht (3) wird üblicherweise auch als Release-Liner bezeichnet. Sie wird üblicherweise auf der haftklebenden Matrixschicht aufgebracht, um ein Verkleben der haftklebenden Matrixschicht beispielsweise mit der Verpackung zu verhindern und vor Gebrauch des transdermalen therapeutischen Systems entfernt. Die Abziehschicht wird bevorzugt aus polymerem Material hergestellt, das gegebenenfalls auch metallisiert sein kann. Beispiele für bevorzugt eingesetzte polymere Materialien sind Polyurethane, Polyvinylacetat, Polyvinylidenchlorid, Polypropylen, Polycarbonat, Polystyrol, Polyethylen, Polyethylenterephthalat, Polybutylenterephthalat sowie gegebenenfalls mit entsprechenden Polymeren oberflächenbeschichtete Papiere. Bevorzugter ist die Abziehschicht einseitig oder beidseitig fluorpolymerbeschichtet oder silikonisiert. Bevorzugt sind handelsübliche fluorpolymerbeschichtete oder silikonisierte Polyesterfolien wie die einseitig silikonisierten Handelsprodukte Primeliner 75 µm oder 100 µm (Firma Loparex, NL) sowie die einseitig fluorpolymerbeschichteten Handelsprodukte Scotchpack 1022 oder Scotchpack 9742 der Firma 3M.

Erfindungsgemäß wesentlich ist die Zusammensetzung der haftklebenden Matrixschicht, die den Wirkstoff enthält. Die für die Matrixschicht verwendeten Polyisobutylene müssen sehr sorgfältig ausgewählt werden, um eine ausreichende und möglichst vollständige Wirkstofffreisetzung zu gewährleisten, gleichzeitig aber auch eine ausreichende Haftung ohne nennenswerte Hautreizungen auf der Haut, über einen Zeitraum von mindestens drei Tagen, und Minimierung von kaltem Fluss auch während der Lagerung sicherzustellen. Die Auswahl der entsprechenden Polymere wird dadurch erschwert, dass die transdermalen therapeutischen Systeme bei einer anderen Temperatur gelagert werden als sie angewendet werden. Die transdermalen therapeutischen Systeme werden bei Raumtemperatur gelagert und dann mit der haftklebenden Schicht auf die menschliche Haut aufgebracht, die eine Temperatur von etwa 32°C aufweist. Bei dieser Temperatur müssen die transdermalen therapeutischen Systeme dann ausreichend haften und eine ausreichende und möglichst vollständige Wirkstofffreisetzung zeigen.

Überraschenderweise wurden erfindungsgemäß transdermale therapeutische Systeme gefunden, bei denen der kalte Fluss insbesondere auch bei der Anwendungstemperatur (Hauttemperatur = 32°C) ausgesprochen niedrig ist, und mit steigender Temperatur fällt. Die erfindungsgemäßen transdermalen therapeutischen Systeme weisen aber auch bei den Temperaturen, bei denen häufig eine Lagerung der transdermalen therapeutischen Systeme stattfindet, einen besonders niedrigen kalten Fluss auf.

Erfindungsgemäß wurde nunmehr überraschend gefunden, dass die Temperaturabhängigkeit des Speichermoduls eines Polyisobutylens einen entscheidenden Einfluss auf die Eigenschaften eines transdermalen therapeutischen Systems hat. Insbesondere wurde gefunden, dass eine hervorragende Wirkstofffreisetzung bis zu einem äußerst geringen Restwirkstoffgehalt bei gleichzeitig exzellenter und langanhaltender Haftung der haftklebenden Matrixschicht auf der Haut und weitgehender Abwesenheit von kaltem Fluss bei der Lagerung des transdermalen therapeutischen Systems erzielt werden kann, wenn als haftklebendes Matrixmaterial ein Gemisch aus zwei Polyisobutylenen verwendet wird, von denen eines, das hier als Polyisobutylen (A) bezeichnet wird im Temperaturbereich von 10 bis 40°C, vorzugsweise von 10 bis 60°C und insbesondere von 0 bis 80°C praktisch keine Temperaturabhängigkeit des Speichermoduls G' zeigt, während das zweite Polyisobutylen, das hier als Polyisobutylen (B) bezeichnet wird in diesem Temperaturbereich ein mit steigender Temperatur kontinuierlich fallendes Speichermodul G' aufweist. Aufgrund dieser überraschenden Erkenntnis wurde dann das erfindungsgemäße transdermale therapeutische System entwickelt.

Das Speichermodul G' sowie das Verlustmodul G" sind dem Fachmann bekannte Parameter, die einen viskoelastischen Stoff kennzeichnen. Man geht davon aus, dass die Stärke des elastischen Anteils eines viskoelastischen Polymers durch das Speichermodul G' und die Stärke des viskosen Anteils eines viskoelastischen Polymers durch das Verlustmodul G" beschrieben wird. Der Quotient aus Verlustmodul G" und Speichermodul G' G"/G' wird als Verlusttangens tan δ bezeichnet. Die Frequenz bei der Messung von G' und G" wird in der vorliegenden Erfindung bei 10 rad/Sekunde konstant gehalten. Im Rahmen der vorliegenden Anmeldung wurde das Speichermodul G' und das Verlustmodul G" mit einer Vorrichtung Rheometrics RDA-III der Firma TA-Instruments, Newcastle, Delaware, USA bestimmt (parallele Platten, 8 mm Durchmesser, Abstand 2-2,5 mm). Die Bestimmung von G' und G" erfolgt bei niedrigen Auslenkspannungen bei der Messung im linearen viskoelastischen Bereich, und ist daher von der Spannung unabhängig. Im Übrigen werden G' und G" nach den Vorschriften der DIN EN ISO 6721-1:2011 bestimmt.

Polyisobutylene sind im Stand der Technik bekannt und werden von einer ganzen Reihe von Firmen angeboten, unter anderem auch in Form von Lösungen. Die Polyisobutylene können übliche Stabilisatoren oder Konservierungsmittel enthalten. Der Ausdruck "Polyisobutylene" wie er im Rahmen dieser Anmeldung verwendet wird, umfasst Polyisobutylene, die derartige übliche Konservierungsmittel und/oder Stabilisatoren enthalten oder nicht enthalten.

Das Speichermodul G' ist neben der Molekulargewichtsverteilung ein typischer Parameter zur Charakterisierung der Polyisobutylene. Polyisobutylene, die durch bestimmte Speichermodule G' gekennzeichnet sind, sind kommerziell erhältlich. Die in den Beispielen der vorliegenden Anmeldung verwendeten Polyisobutylenlösungen wurden von der Firma Henkel Corporation, Bridgewater, USA erhalten, es können aber selbstverständlich auch Konkurrenzprodukte verwendet werden. Die Angabe des gewünschten Verlaufs des Speichermoduls ist für den Hersteller der Polyisobutylene ausreichend, um ein entsprechendes Polyisobutylen zur Verfügung zu stellen. Selbstverständlich wird das Speichermodul an dem Polyisobutylen nach Entfernen der Lösemittel bestimmt.

Das erfindungsgemäße transdermale therapeutische System weist eine haftklebende Matrixschicht auf, die ein Gemisch aus einem Polyisobutylen A und einem Polyisobutylen B enthält.

Das Polyisobutylen A ist durch sein Speichermodul G' gekennzeichnet, das im Temperaturbereich von 10 bis 40°C, vorzugsweise von 10 bis 60°C und insbesondere von 0 bis 80°C im Wesentlichen konstant ist, das heißt das Speichermodul G' weist in diesem Temperaturbereich ein Plateau auf. Unter "im Wesentlichen konstant" wird erfindungsgemäß verstanden, dass sich bei der Auftragung der Werte des Speichermoduls G' (als Logarithmus) gegen die Temperatur im Temperaturbereich von zumindest 10 bis 40°C, vorzugsweise von 10 bis 60°C und insbesondere von 0 bis 80°C ein flaches Plateau zeigt. In einer bevorzugten Ausführungsform des erfindungsgemäßen transdermalen therapeutischen Systems bedeutet "im Wesentlichen konstant", dass in dem angegebenen Temperaturbereich keiner der Werte für das Speichermodul G' mehr als 50% (absolut, d.h. nicht logarithmisch), stärker bevorzugt mehr als 25% (absolut) und insbesondere mehr als 15% (absolut) von dem Wert des Speichermoduls bei 40°C, nach unten abweicht und dass keiner der Werte für das Speichermodul G' mehr als 100% (absolut), bevorzugt mehr als 50% (absolut), stärker bevorzugt mehr als 25% (absolut) nach oben abweicht.

Der Absolutwert des Plateaus im Bereich von 10 bis 40°C, vorzugsweise von 10 bis 60°C und insbesondere von 0 bis 80°C liegt für das Polyisobutylen A vorzugsweise in dem Bereich von 5 × 10⁴ Pascal bis 5 × 10⁶ Pascal, insbesondere im Bereich von 10⁵ bis 10⁶ Pascal und am stärksten bevorzugt im Bereich von 10⁵ Pascal bis 5 × 10⁵ Pascal. Die Molekulargewichtsverteilung des Polyisobutylens A ist für die vorliegende Erfindung nicht relevant, so lange die Werte für das Speichermodul G' eingehalten werden.

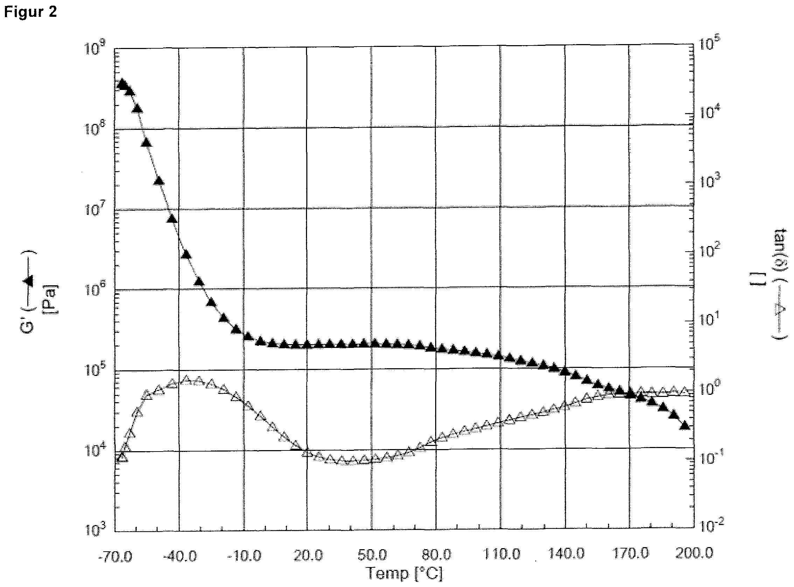

Das Polyisobutylen A kann beispielsweise durch Mischen von zwei oder mehr Polyisobutylenen hergestellt werden, so dass sich ein Polyisobutylen mit dem gewünschten Verlauf für das Speichermodul ergibt. Vorzugsweise handelt es sich bei dem Polyisobutylen A jedoch um ein einzelnes Polyisobutylen. Ein Beispiel für ein geeignetes Polyisobutylen A ist das Produkt Durotak 87-625A der Firma Henkel Corporation, Brigewater, USA. Der Verlauf des Speichermoduls G' (zusammen mit dem Verlauf des Quotienten aus dem Verlustmodul G" und dem Speichermodul G', der als tan δ oder auch als Verlusttangens bezeichnet wird) für das kommerzielle Produkt Durotak 87-625A ist in Figur 2 gezeigt.

Während das Produkt Durotak 87-625A als Polyisobutylen A gemäß der vorliegenden Erfindung bevorzugt ist, können selbstverständlich auch entsprechende Produkte anderer Hersteller oder des gleichen Herstellers verwendet werden, bei denen die Temperaturabhängigkeit des Speichermoduls im erfindungsgemäßen Bereich liegt.

In der haftklebenden Matrixschicht des erfindungsgemäßen transdermalen therapeutischen Systems liegt das Polyisobutylen A im Gemisch mit einem zweiten Polyisobutylen vor, das hier als Polyisobutylen B bezeichnet wird.

Auch das Polyisobutylen B wird durch sein Speichermodul G' gekennzeichnet. Anders als bei dem Polyisobutylen A muss bei dem Polyisobutylen B das Speichermodul im Bereich von 10 bis 40°C, vorzugsweise von 10 bis 60°C und insbesondere von 0 bis 80°C kontinuierlich fallen, und der Wert für das Speichermodul G' des Polyisobutylens B ist bei der niedrigeren Temperatur des jeweiligen Bereichs deutlich höher als bei der höheren Temperatur des jeweiligen Bereichs. Das Speichermodul G' des Polyisobutylens B ist in der Regel bei 0°C mindestens doppelt so hoch (absolut) wie bei 80°C, bevorzugt ist das Speichermodul G' bei 0°C mindestens fünf Mal so hoch (absolut) wie bei 80°C und besonders bevorzugt ist das Speichermodul G' des Polyisobutylens B bei 0°C mindestens 10 Mal so hoch (absolut) wie bei 80°C. In der Regel ist auch bei dem Polyisobutylen B das Speichermodul G' bei einer Temperatur von 10°C mindestens doppelt so hoch (absolut), bevorzugt mindestens dreimal so hoch (absolut) wie bei einer Temperatur von 40°C.

Der Absolutwert des Speichermoduls G' bei 0°C ist bei dem Polyisobutylen B bevorzugt im Bereich von 5 × 10⁴ Pascal bis 5 × 10⁶ Pascal, insbesondere im Bereich von 5 × 10⁴ Pascal bis 10⁶ Pascal und ganz besonders bevorzugt im Bereich von 10⁵ bis 10⁶ Pascal. Der Absolutwert des Speichermoduls G' bei 80°C ist bei dem Polyisobutylen bevorzugt im Bereich von 5 × 10² Pascal bis 5 × 10⁴ Pascal, stärker bevorzugt im Bereich von 5 × 10² Pascal bis 10⁴ Pascal und insbesondere im Bereich von 10³ bis 10⁴ Pascal.

Der Absolutwert des Speichermoduls G' ist bei den Polyisobutylen B bei 10°C, bevorzugt im Bereich von 2×10⁴ Pascal bis 10⁶ Pascal, insbesondere im Bereich von 5×10⁴ Pascal bis 10⁶ Pascal. Der Absolutwert des Speichermoduls G' bei 40°C ist bei dem Polyisobutylen B bevorzugt im Bereich von 5×10³ Pascal bis 2×10⁵ Pascal, stärker bevorzugt im Bereich von 10⁴ Pascal bis 10⁵ Pascal. Der Absolutwert des Speichermoduls G' bei 60°C ist bei dem Polyisobutylen B bevorzugt im Bereich von 5×10² Pascal bis 10⁵ Pascal, stärker bevorzugt im Bereich von 5×10³ Pascal bis 5×10⁴ Pascal. Wie beim Polyisobutylen A ist auch für das Polyisobutylen B die Molekulargewichtsverteilung für die vorliegende Erfindung nicht relevant, so lange das Speichermodul G' die erfindungsgemäß erforderliche Temperaturabhängigkeit aufweist.

Weiterhin hat sich gezeigt, dass zur Lösung der erfindungsgemäßen Ausgabe derartige transdermale therapeutische Systeme bevorzugt sind, die ein bestimmtes Verhältnis von Speichermodul zu Verlustmodul aufweisen. Bei dieser bevorzugten Ausführungsform können die erfindungsgemäßen transdermalen therapeutischen Systeme zusätzlich durch den Wert des Verlusttangens δ bei 30°C angegeben werden. So ist bei dem Polyisobutylen A der Verlusttangens δ bei 30°C, vorzugsweise im Bereich von 10⁻² bis 5×10⁻¹, stärker bevorzugt im Bereich von 5×10⁻² bis 5×10⁻¹. Bei den erfindungsgemäßen Polyisobutylenen B ist der Verlusttangens δ bei 30°C bevorzugt im Bereich von mehr als 5×10⁻¹ bis 10, insbesondere im Bereich von 6×10⁻¹ bis 5. Bevorzugt ist also in erfindungsgemäßen transdermalen therapeutischen Systemen der Verlusttangens bei dem Polyisobutylen B bei 30°C höher als bei dem Polyisobutylen A.

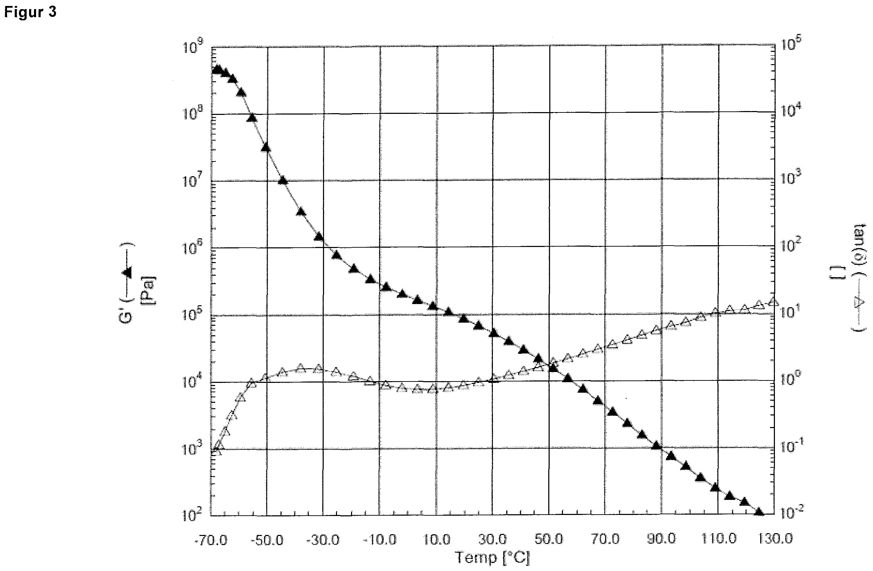

Wie das Polyisobutylen A kann auch das Polyisobutylen B beispielsweise durch Mischen von zwei oder mehr Polyisobutylenen hergestellt werden, so dass sich ein Polyisobutylen mit dem gewünschten Verlauf für das Speichermodul G' ergibt. Vorzugsweise handelt es sich bei Polyisobutylen B um ein einzelnes Polyisobutylen. Ein Beispiel für ein geeignetes Polyisobutylen B ist das Produkt Durotak 87-626A der Firma Henkel Corporation, Bridgewater, USA. Der Verlauf des Speichermoduls G' (zusammen mit dem Verlauf des Quotienten aus dem Verlustmodul G" und dem Speichermodul G', der als tan δ (oder auch als Verlusttangens) bezeichnet wird), für das kommerzielle Produkt Durotak 87-626A ist in Figur 3 gezeigt.

Während das Produkt Durotak 87-626A als Polyisobutylen B gemäß der vorliegenden Erfindung bevorzugt ist, können selbstverständlich auch entsprechende Produkte anderer Hersteller oder des gleichen Herstellers verwendet werden, bei denen die Temperaturabhängigkeit des Speichermoduls im erfindungsgemäßen Bereich liegt.

Da die haftklebende Matrixschicht des erfindungsgemäßen transdermalen therapeutischen Systems ein Gemisch des Polyisobutylens A und des Polyisobutylens B enthält und das Polyisobutylen A und/oder das Polyisobutylen B bevorzugt aus jeweils einem Polyisobutylen oder jeweils aus einem Gemisch aus zumindest zwei Polyisobutylenen mit unterschiedlichem mittleren Molekulargewicht besteht, kann die haftklebende Matrix des erfindungsgemäßen transdermalen therapeutischen Systems zwei, drei, vier oder mehr Polyisobutylene mit unterschiedlichem mittleren Molekulargewicht aufweisen.

Sofern im Rahmen der vorliegenden Beschreibung von einem mittleren Molekulargewicht die Rede ist, wird hierunter ein gewichtsgemitteltes mittleres Molekulargewicht verstanden, sofern nichts anderes ausdrücklich erwähnt ist oder sich aus den Umständen ergibt.

Erfindungsgemäß wird davon ausgegangen, dass ein einzelnes Polyisobutylen eine Molekulargewichtsverteilung mit einem einzelnen Peak ohne Schulter aufweist, die derjenigen Molekulargewichtsverteilung entspricht, die man bei der Polymerisation des Polyisobutylens aus den einzelnen Monomeren erhält. Werden zwei durch einfache Polymerisation hergestellte Polyisobutylene unterschiedlichen Molekulargewichts gemischt, liegt erfindungsgemäß ein Gemisch aus zwei Polyisobutylenen vor. Ein Gemisch aus zwei Polyisobutylenen ist erfindungsgemäß dadurch gekennzeichnet, dass die Molekulargewichtsverteilung des Gemisches aus zwei Polyisobutylenen zwei Peaks bei zwei unterschiedlichen Molekulargewichten aufweist oder die Molekulargewichtsverteilung einen Peak mit einer Schulter zeigt (falls die mittleren Molekulargewichte der gemischten Polyisobutylene zu nahe aneinander liegen, so dass sich keine zwei getrennten Peaks zeigen).

Bei dem erfindungsgemäßen transdermalen therapeutischen System besteht die haftklebende Matrixschicht aus einem Gemisch aus einem Polyisobutylen A und einem Polyisobutylen B, die sich durch die Temperaturabhängigkeit ihrer Speichermodule unterscheiden. In der Regel weist das Polyisobutylen A auch ein anders mittleres Molekulargewicht auf als das Polyisobutylen B. In der Regel wird daher die Molekulargewichtsverteilung des Gemisches aus dem Polyisobutylen A und dem Polyisobutylen B mindestens zwei Peaks aufweisen, von denen einer auf das Polyisobutylen A und einer auf das Polyisobutylen B zurückgeht. Es ist auch möglich, dass das Polyisobutylen A und/oder das Polyisobutylen B wiederum aus mindestens zwei Polyisobutylenen mit unterschiedlichem Molekulargewicht hergestellt wurde. In dem erfindungsgemäßen transdermalen therapeutischen System ist die haftklebende Matrixschicht aber bevorzugt aus zwei Polyisobutylenen aufgebaut, wobei jedes Polyisobutylen in der haftklebenden Matrixschicht des erfindungsgemäßen transdermalen therapeutischen Systems einen gesonderten Peak oder eine Schulter in der Molekulargewichtsverteilung zeigt.

Für den Fall, dass es sich bei Polyisobutylen A oder B jeweils um Gemische aus Polyisobutylen handelt, werden die einzelnen Polyisobutylene, aus denen das Polyisobutylen A und das Polyisobutylen B besteht, so gewählt, dass man jeweils die gewünschte Temperaturabhängigkeit des Speichermoduls für Polyisobutylen A bzw. B erhält.

Die Molekulargewichtsverteilung eines Polyisobutylens bzw. eines Gemisches mehrerer Polyisobutylene kann im Stand der Technik durch Gelpermeations-Chromatographie (GPC), beispielsweise gegen einen Polystyrol-Standard, bestimmt werden.

Die Menge an Polyisobutylen B in der haftklebenden Matrixschicht im Verhältnis zur Menge an Polyisobutylen A in der haftklebenden Matrixschicht kann von einem Fachmann geeignet eingestellt werden. Gemäß Erfindung liegt das Gewichtsverhältnis von Polyisobutylen A : Polyisobutylen B in der Matrixschicht im Bereich von 20% (A) : 80% (B) bis 40% (A) : 60% (B) und insbesondere 25% (A) : 75% (B) bis 35% (A) : 65% (B), jeweils bezogen auf das Gesamtgewicht der beiden Polyisobutylene, und bevorzugt ist aber der Gehalt an Polyisobutylen B in der Matrixschicht höher als der Gehalt an Polyisobutylen A. Die Menge an Polyisobutylen A in der Matrixschicht ist bevorzugt im Bereich von 10 bis 25%, stärker bevorzugt im Bereich von 10 bis 20%, insbesondere im Bereich von 12 bis 20%, insbesondere von 16 bis 18%, jeweils als Gewichtsprozent bezogen auf das Gesamtgewicht der Matrixschicht. Der Gehalt an Polyisobutylen B in der Matrixschicht liegt bevorzugt im Bereich von 30 bis 60%, stärker bevorzugt im Bereich von 30 bis 50% und insbesondere im Bereich von 40 bis 45%, jeweils als Gewichtsprozent bezogen auf das Gesamtgewicht der Matrixschicht.

Die Matrixschicht des erfindungsgemäßen transdermalen therapeutischen Systems enthält den Wirkstoff sowie das Polyisobutylen A und das Polyisobutylen B. Weiterhin enthält die Matrixschicht bevorzugt noch einen Permeationsverstärker. Als Permeationsverstärker kann ein beliebiger im Stand der Technik bekannter Permeationsverstärker für den Wirkstoff Fentanyl verwendet werden. Besonders bevorzugt wird erfindungsgemäß ein Carbonsäureester und hier insbesondere ein Fettsäureester verwendet. Besonders bevorzugt sind der Myristinsäureisopropylester (Isopropylmyristat) und der Ölsäureoleylester (Oleyloleat). Überraschenderweise hat sich gezeigt, dass bei Verwendung der erfindungsgemäßen Matrix der Permeationsverstärker Isopropylmyristat besonders gut geeignet ist, um ein transdermales therapeutisches System mit den gewünschten Eigenschaften bereitzustellen.

Der Gehalt an Permeationsverstärker in der Matrixschicht des erfindungsgemäßen transdermalen therapeutischen Systems ist nicht besonders eingeschränkt, er liegt aber in der Regel im Bereich von 2 bis 20 Gew.-% bezogen auf das Gesamtgewicht der Matrixschicht, besonders bevorzugt im Bereich von 5 bis 15 Gew.-%, beispielsweise bei 10 Gew.-% bezogen auf das Gesamtgewicht der Matrixschicht. Bei Verwendung von Isopropylmyristat als Permeationsverstärker hat sich der Bereich von 8 bis 15 Gew.-%, bezogen auf das Gesamtgewicht der erfindungsgemäßen Matrixschicht, als besonders vorteilhaft herausgestellt.

Weiterhin bevorzugt enthält die haftklebende Matrixschicht des erfindungsgemäßen transdermalen therapeutischen Systems auch noch einen Klebrigmacher. Als Klebrigmacher eignet sich insbesondere ein Polybuten, es kann aber auch ein anderer für Polyisobutylene geeigneter Klebrigmacher wie Rosinharz, Terpenharz oder Petrolharz verwendet werden. Polybuten ist als Klebrigmacher jedoch bevorzugt, insbesondere Polybuten mit einem zahlengemittelten Molekulargewicht im Bereich von 700 bis 6000, insbesondere von 900 bis 4000, wie das Produkt Indopol H-1900 mit einem zahlengemittelten mittleren Molekulargewicht von 2500. Bei Polybuten handelt es sich um ein Isobutylen/buten Copolymer.

Weiterhin ist als Klebrigmacher ein hydriertes oder nicht-hydriertes Kolophoniumharz, insbesondere ein hydriertes Kolophoniumharz, bevorzugt. Als Beispiel hierfür kann das kommerzielle Produkt Foral^{®} 105-E der Firma Eastman Chemical Middelburg BV, Den Haag, Niederlande genannt werden.

Der Gehalt an Klebrigmacher, insbesondere an Polybuten in der Matrixschicht des erfindungsgemäßen transdermalen therapeutischen Systems beträgt in der Regel 0 bis 40 Gew.-%, vorzugsweise 5 bis 35 Gew.-%, insbesondere 10 bis 30 Gew.-%, z.B. etwa 25 Gew.-%, jeweils bezogen auf das Gesamtgewicht der Matrixschicht.

Bei Verwendung von Isopropylmyristat als Permeationsverstärker hat es sich überraschenderweise als vorteilhaft herausgestellt, ein Polybuten mit einem zahlengemittelten Molekulargewicht im Bereich von 1800 bis 2800 zu verwenden, und zwar besonders vorteilhaft im Bereich von 22 bis 28 Gew.-%, bezogen auf das Gesamtgewicht der erfindungsgemäßen Matrixschicht.

Überraschenderweise hat sich zudem gezeigt, dass bei den erfindungsgemäß bevorzugten Mengenverhältnissen von Indopol 1900, Polyisobutylen A und Polyisobutylen B in dem transdermalen therapeutischen System der kalte Fluss bei typischen Lagertemperaturen von weniger als 20°C und nach Aufbringen auf die Haut (d.h. bei Temperaturen >30°C) besonders gering ist.

Weitere Bestandteile befinden sich erfindungsgemäß bevorzugt nicht in der Matrixschicht des erfindungsgemäßen transdermalen therapeutischen Systems. Die Matrixschicht des erfindungsgemäßen transdermalen therapeutischen Systems besteht daher erfindungsgemäß bevorzugt aus Wirkstoff, Polyisobutylen A, Polyisobutylen B, Permeationsverstärker (insbesondere ein Carbonsäureester, bevorzugt Isopropylmyristat oder Oleyloleat, besonders bevorzugt Isopropylmyristat) und Klebrigmacher (besonders bevorzugt Polybuten, insbesondere Indopol H-1900).

Die Matrixschicht der erfindungsgemäßen transdermalen therapeutischen Systeme ist insbesondere frei von Gelbildnern.

Die Matrix der erfindungsgemäßen transdermalen therapeutischen Systeme weist bevorzugt keinen verzweigten höherkettigen Alkohol, insbesondere bevorzugt überhaupt keinen höherkettigen Alkohol auf, wobei unter einem verzweigten höherkettigen Alkohol oder einem höherkettigen Alkohol ein Alkohol mit 5 oder mehr Kohlenstoffatomen verstanden wird.

In einer bevorzugten Ausführungsform weist das Polyisobutylen A beispielsweise ein viskositätsgemitteltes mittleres Molekulargewicht von ca. 1,100,000 g/mol und das Polyisobutylen B ein viskositätsgemitteltes mittleres Molekulargewicht von ca. 30.000 bis ca. 60.000 g/mol, vorzugsweise ca. 40.000 oder 55.000 g/mol auf. Bei dieser Ausführungsform enthält das Pflaster weiterhin einen Klebrigmacher und einen Permeationsverstärker, insbesondere den Klebrigmacher Indopol H 1900, also ein Polybuten mit einem zahlengemittelten Molekulargewicht von etwa 2500 g/mol und als Permeationsverstärker insbesondere entweder Oleyloleat oder Isopropylmyristat, wobei das Polybuten bevorzugt in einer Menge von 23 bis 28 Gew.-% vorhanden ist und das Isopropylmyristat bzw. das Oleyloleat in einer Menge von 8 bis 15 Gew.-% vorhanden ist.

In dem transdermalen therapeutischen System gemäß der Erfindung weist die Matrixschicht eine Menge an Fentanyl oder einem Analogstoff davon auf, die ausreichend ist, um Schmerzfreiheit bei einem Menschen zu induzieren und für drei bis sieben Tage aufrechtzuerhalten (bezogen auf den Zeitpunkt der Verabreichung des Pflasters).

Die absolute Menge an einzusetzendem Wirkstoff hängt von verschiedenen Faktoren, insbesondere von der Größe des einzusetzenden Pflasters und der Anwendungsdauer ab. Bevorzugt enthält das transdermale therapeutische System den Wirkstoff (insbesondere Fentanyl) in einer Menge von 3 bis 15 Gew.-%, besonders bevorzugt von 3 bis 10 Gew.- %, insbesondere von 4 bis 8 Gew.-%, 4 bis 6 Gew.-% oder 5 bis 6 Gew.-%, beispielsweise von etwa 6 Gew.-%, jeweils bezogen auf das Gesamtgewicht der Matrixschicht. In Abhängigkeit von der genauen Zusammensetzung der Matrixschicht und der verwendeten Menge an Wirkstoff kann der Wirkstoff entweder vollständig gelöst in der Matrixschicht vorliegen, oder es befinden sich einzelne Wirkstoffteilchen ungelöst in der Matrixschicht. Erfindungsgemäß befinden sich einzelne Wirkstoffteilchen ungelöst in der Matrixschicht. In dem Fall, spricht man von einem Suspensionspflaster. Ein erfindungsgemäßes Suspensionspflaster ist in Figur 1 gezeigt, wobei Bezugszeichen 4 die festen Wirkstoffteilchen in der Matrixschicht kennzeichnet.

Aus den vorstehenden Ausführungen ergeben sich bevorzugte Ausgestaltungen des erfindungsgemäßen transdermalen therapeutischen Systems, bei denen die Matrixschicht eine der in der nachstehenden Tabelle zusammengefassten Zusammensetzung aufweist.

| **BESTANDTEIL** | **GEW-%** | **GEW-%** | **GEW-%** | **GEW-%** | **GEW-%** |
|---|---|---|---|---|---|
| **Fentanyl** | 3 - 10 | 4 - 8 | 4 - 6 | 5.0 - 6.0 | 6 |
| **Permeationsverstärker** | 5 - 15 | 8 - 15 | 8 - 12 | 9 - 11 | 10 |
| **Polyisobutylen B** | 20 - 60 | 30 - 50 | 35 - 50 | 40 - 45 | 41,2 |
| **Polyisobutylen A** | 5 - 30 | 8 - 25 | 12 - 20 | 16 - 18 | 17,6 |
| **Klebrigmacher** | 15 - 45 | 15 - 40 | 20 - 35 | 23 - 28 | 25,2 |

| **BESTANDTEIL** | **GEW-%** | **GEW-%** | **GEW-%** | **GEW-%** | **GEW-%** |
|---|---|---|---|---|---|
| **Fentanyl** | 3 - 10 | 4 - 8 | 4 - 6 | 5.0 - 6.0 | 6 |
| **Isopropylmyristat** | 5 - 15 | 8 - 15 | 8 - 12 | 9 - 11 | 10 |
| **Polyisobutylen B** (besonders bevorzugt Durotak 87-626A) | 20 - 60 | 30 - 50 | 35 - 50 | 40 - 45 | 41,2 |
| **Polyisobutylen A** (besonders bevorzugt Durotak 87-625A) | 5 - 30 | 8 - 25 | 12 - 20 | 16 - 18 | 17,6 |
| **Polybuten** mit einem zahlengemittelten Molekulargewicht von etwa 2500 | 15 - 45 | 15 - 40 | 20 - 35 | 23 - 28 | 25,2 |

Die Gewichtsprozentangaben in den vorstehenden Tabellen beziehen sich jeweils auf das Gesamtgewicht der Matrixschicht. Das Flächengewicht der Matrixschicht liegt erfindungsgemäß bevorzugt im Bereich von 20 g/m² bis 100 g/m², insbesondere im Bereich von 30 g/m² bis 70 g/m², jeweils bezogen auf das Flächengewicht der getrockneten Matrixschicht.

Die vorliegende Erfindung betrifft ein transdermales System zur Linderung von Schmerz während der vorgesehenen Tragezeit, wie oben beschrieben, bei dem die Matrixschicht, nach Applikation auf der Haut für die Dauer der vorgesehenen Tragezeit von 3 bis 7 Tagen, insbesondere 3 Tagen, einen Restwirkstoffgehalt von unter 35%, vorzugsweise unter 25%, mehr bevorzugt unter 20%, noch mehr bevorzugt unter 15%, insbesondere unter 10%, z.B. unter 8%, 7%, 6% oder 5% des Ausgangsgehaltes an Wirkstoff aufweist. Typischerweise liegt der Restwirkstoffgehalt im Bereich von unter 35% bis 10%, bevorzugt unter 25% bis 2%, mehr bevorzugt im Bereich von unter 20% bis 3%, noch mehr bevorzugt im Bereich von unter 15% bis 4%, insbesondere unter 10% bis 5% des Ausgangsgehalts an Wirkstoff. Der Ausgangsgehalt an Wirkstoff bezieht sich auf die absolute Menge an einzusetzendem Wirkstoff in der Matrixschicht wie oben definiert. Der Restwirkstoffgehalt (in %) ergibt sich aus dem Quotient aus der Absolutmenge an Restwirkstoffgehalt der Matrixschicht und der absoluten Menge an eingesetztem Wirkstoff. Die Absolutmenge an Restwirkstoffgehalt der Matrixschicht ist die im transdermalen System nach Applikation auf der Haut für die vorgesehene Tragezeigt, von 3 bis 7 Tagen, z.B. 3 Tage, verbleibende Menge an Wirkstoff. Die Absolutmenge an Restwirkstoff eines transdermalen Systems kann mit im Stand der Technik bekannten Methoden bestimmt werden. So kann beispielsweise die Matrixschicht des gebrauchten transdermalen Systems in einem geeigneten Lösungsmittel aufgelöst werden, und die Gesamtmenge an Wirkstoff in der Lösung bestimmt werden, beispielsweise chromatographisch.

Es hat sich überraschenderweise gezeigt, dass insbesondere durch die Wahl der oben angegebenen Polymere in der Matrixschicht trotz geringem Wirkstoffeinsatz ein ausgezeichneter Wirkstofffluss und damit hohe Abgaberaten über den vorgesehenen Applikationszeitraum des transdermalen Systems bis hin zu äußerst niedrigen Restwirkstoffgehalten realisiert werden kann. Bevorzugt weist das transdermale therapeutische System gemäß der vorliegenden Erfindung eine Abgaberate des Wirkstoffs auf, die der eines transdermalen therapeutischen Systems entspricht, dass von mindestens einer Arzneimittelbehörde zugelassen wurde. Typische derartige Abgaberaten sind im Bereich von etwa 12,5 µg/h bis etwa 100 µg/h, vorzugsweise 12,5 µg/h, 25 µg/h, 50 µg/h, 75 µg/h und/oder 100 µg/h. In einer Ausführungsform weist das transdermale therapeutische System eine Abgaberate von mindestens 100 µg/h oder mehr auf, vorzugsweise mindestens 100 µg/h oder mehr bis etwa 300 µg/h, insbesondere etwa 150 µg/h bis etwa 250 µg/h, z.B. etwa 200 µg/h oder 250 µg/h. Vorzugsweise werden die Abgaberaten bei einer im Vergleich zu bekannten, insbesondere zugelassene transdermale therapeutische Systeme geringer Wirkstoffbeladung (Absolutmenge an Wirkstoff im transdermalen therapeutischen System) erreicht. Durch die erfindungsgemäß erreichten niedrige Restwirkstoffgehalt bei hohen Abgaberaten werden somit nicht nur unnötige Kosten durch im transdermalen System verbleibenden ungenutzten Wirkstoff vermieden, sondern auch das Gefahr- und Missbrauchspotenzial durch getragene und noch Restwirkstoffgehalt aufweisende transdermale System vermindert. Die vorliegende Erfindung betrifft daher auch getragene transdermale therapeutische Systeme wie oben definiert mit dem oben angegebenen Gehalt an Restwirkstoff nach Gebrauch.

Das erfindungsgemäße transdermale therapeutische System wird nach im Stand der Technik im Prinzip bekannten Verfahren hergestellt. Hierzu wird der Wirkstoff im Permeationsverstärker dispergiert. Das Polyisobutylen A und das Polyisobutylen B werden ebenfalls in einem geeigneten Lösemittel insbesondere einem flüssigen Alkan, insbesondere einen Alkan mit 5 bis 7 Kohlenstoffatomen, wie z.B. Heptan gelöst. Die gelösten Polyisobutylene werden unter Rühren mit dem dispergierten Fentanyl gemischt. Anschließend wird gegebenenfalls der Klebrigmacher zugegeben und es wird weitergerührt, bis eine homogene Beschichtungsmasse entsteht. Die homogene Beschichtungsmasse wird dann auf die Abziehschicht aufgetragen und unter Erwärmen getrocknet, so dass der Restlösemittelgehalt möglichst gering ist. Bevorzugt beträgt der Restlösemittelgehalt weniger als 1%, insbesondere weniger als 0,5%. Die getrocknete Matrix wird mit der Rückschicht kaschiert, wodurch ein Laminat entsteht. Aus diesem Laminat werden Pflaster geeigneter Größe ausgestanzt.

Die hier beschriebenen transdermalen therapeutischen Systeme können nach dem vorstehenden erfindungsgemäßen Verfahren hergestellt werden und die Erfindung betrifft daher auch das Verfahren zur Herstellung der erfindungsgemäßen transdermalen therapeutischen Systeme und die nach diesem Verfahren erhältlichen transdermalen therapeutischen Systeme.

Desweiteren betrifft die vorliegende Erfindung auch die Verwendung eines Gemischs aus Polyisobutylen A und Polyisobutylen B wie oben beschrieben zur Herstellung einer Matrixschicht eines transdermalen therapeutischen Systems oder die Verwendung einer derartigen Matrixschicht zur Verabreichung von Fentanyl oder eines Analogstoffes des Fentanyl wie oben beschrieben, wobei die Matrixschicht des transdermalen therapeutischen Systems nach Applikation auf der Haut für die vorgesehene Tragezeit von 3 bis 7 Tagen, insbesondere 3 Tagen, einen Restwirkstoffgehalt wie oben für die Matrix definiert, unter 35%, vorzugsweise unter 25%, mehr bevorzugt unter 20%, noch mehr bevorzugt unter 15%, insbesondere unter 10%, zum Beispiel unter 8%, 7%, 6% oder 5% des Ausgangsgehalts an Wirkstoff aufweist. Auch betrifft die vorliegende Erfindung die Verwendung eines derartigen Gemischs aus Polyisobutylen A und Polyisobutylen B zur Herstellung einer Matrixschicht eines transdermalen therapeutischen Systems oder die Verwendung einer derartigen Matrixschicht in einem transdermalen therapeutischen System, das, insbesondere nach Gebrauch, gegen Missbrauch oder Fehlgebrauch geschützt ist. Auch betrifft die vorliegende Erfindung die Verwendung eines Gemischs aus Polyisobutylen A und Polyisobutylen B zur Verkleinerung der Größe eines transdermalen therapeutischen Systems bei im Wesentlich gleichbleibender Freisetzung, insbesondere den oben genannten Abgaberaten. Insbesondere wird erfindungsgemäß ein Gemisch aus Polyisobutylen A und Polyisobutylen B zur Herstellung einer Matrixschicht eines transdermalen therapeutischen Systems verwendet oder eine derartige Matrixschicht in einem transdermalen therapeutischen System eingesetzt, um die Größe des transdermalen therapeutischen Systems bei im Vergleich zu einem kommerziell erhältlichen transdermalen therapeutischen System, wie zum Beispiel Matrifen^{®} und Durogesic DTrans^{®} bei gleichbleibenden, also bioequivalentem Freisetzungsprofil zu verkleinern. Damit wird erfindungsgemäß ein transdermales therapeutisches System zur Verfügung gestellt, das bei geringer Größe zum Marktprodukt, insbesondere dem kommerziell erhältlichen Matrifen^{®} oder Durogesic DTrans^{®}, bioequivalent ist. Der Einsatz kleiner transdermaler therapeutischer Systeme ist insbesondere aus kosmetischen Gründen wünschenswert.

Schließlich betrifft die vorliegende Erfindung die Verwendung eines Gemisches aus Polyisobutylen A und Polyisobutylen B wie oben beschrieben zur Herstellung einer Matrixschicht eines transdermalen therapeutischen Systems oder die Verwendung einer derartigen Matrixschicht in einem transdermalen therapeutischen System, das eine Abgaberate wie oben definiert, insbesondere von mindestens 200 µg/h, z.B. mindestens 250 µg/h aufweist. Vorzugsweise weist das erfindungsgemäße transdermale therapeutische System eine Größe von weniger als 80 cm², mehr bevorzugt weniger als 60 cm², noch mehr bevorzugt weniger als 50 cm², insbesondere weniger als 45 cm² auf.

Das folgende Beispiel erläutert die Erfindung.

Ein transdermales therapeutisches System mit einer haftklebenden Matrixschicht der folgenden Zusammensetzung:
- 6% Fentanyl
- 10% Isopropylmyristat
- 41,2% Durotak 87-626A
- 17,6% Durotak 87-625A und
- 25,2% Indopol 1900
wurde wie folgt hergestellt.

0,6 g Fentanyl wurden in 1,0 g Isopropylmyristat dispergiert. Der Ansatz wurde bis zur homogenen Suspension gerührt. 5,58 g Durotak 87-626A (Feststoffgehalt 73,71%) und 14,48 g Durotak 87-625A (Feststoffgehalt 12,18%) wurden unter Rühren über einen Zeitraum von 20 Minuten zu dem Ansatz aus Fentanyl und Isopropylmyristat zugegeben. Anschließend wurden 2,52 g Polybuten zu dem restlichen Gemisch zugefügt.

Es wurde gerührt, bis eine homogene Beschichtungsmasse entsteht. Diese homogene Beschichtungsmasse wurde auf einer Folie der Marke Scotchpak 9742 in einer Dicke von 117 µm aufgetragen. Das Gemisch wurde anschließend stufenweise auf 50°C, 60°C, 70°C und 80°C erwärmt und getrocknet. Das Flächengewicht der getrockneten Matrixschicht beträgt ca. 50 g/m². Die getrocknete Matrix wurde mit einer Rückschicht der Marke Scotchpak 9723 kaschiert. Aus dem Laminat wurden Pflasterfragmente in geeigneter Größe ausgestanzt.

Die so hergestellten Pflaster wurden 8 Wochen im Kühlschrank bei 2°C bis 8°C, bei 25°C und 60% relativer Luftfeuchtigkeit und bei 40°C und 75% relativer Luftfeuchtigkeit gelagert. Bei den Pflastern trat kein nennenswerter kalter Fluss auf. Bei allen untersuchten Pflastern unter allen untersuchten Bedingungen lag der maximale kalte Fluss unter 0,3 mm. Als Vergleich wurde ein transdermales therapeutisches System gemäß Beispiel 2 der WO 2011/029948 hergestellt und über 8 Wochen bei 25°C/60% Luftfeuchtigkeit und bei 40°C/75% Luftfeuchtigkeit gelagert. Die Vergleichspflaster zeigten einen ausgeprägten kalten Fluss an der Abziehhilfe und auch an der Rückschicht, der wesentlich größer war als der kalte Fluss, der sich bei dem erfindungsgemäßen Pflaster zeigte.

Mit den erfindungsgemäßen Pflastern wurden *in* vitro-Studien durchgeführt. Es zeigten sich Permeationsraten in Franz-Zellen, die im Wesentlichen denen des Pflasters des Beispiels 2 der WO 2011/029948 entspricht.

Die Pflaster haften ohne nennenswerte Hautreizungen und werden lokal bis zu drei Tagen auf der Haut gut vertragen.

Damit zeigen die erfindungsgemäßen transdermalen therapeutischen Systeme die gleiche gute Hautverträglichkeit, die gleiche Klebkraft und gleiche Permeationsraten wie gängige kommerzielle Systeme. Anders als die transdermalen therapeutischen Systeme der WO 2011/029948 zeigen die erfindungsgemäßen transdermalen therapeutischen Systeme aber bei der Lagerung praktisch keinen kalten Fluss und sind auch wesentlich einfacher und kostengünstiger mit noch geringerem Wirkstoffgehalt und geringen Restwirkstoffgehalt herzustellen.

## Patentansprüche

1. Transdermales therapeutisches System zur Verwendung zur Linderung von Schmerz durch Verabreichung eines Wirkstoffs durch die Haut während einer vorgesehenen Tragezeit von 3 bis 7 Tagen bestehend aus
a) einer Rückschicht,
b) einer haftklebenden Matrixschicht, die den Wirkstoff enthält,
und
c) einer Abziehschicht (Release-Liner),
sowie gegebenenfalls
b1) einer zusätzlichen Klebstoffschicht oder einem Overtape, die die Haftung des transdermalen therapeutischen Systems auf der Haut verbessern und die auf der haftklebenden Matrixschicht aufgebracht sind, oder
b2) einer Membran, die die Freisetzung des Wirkstoffes kontrolliert und auf der sich eine Klebstoffschicht befindet, wobei die Membran auf der haftklebenden Matrixschicht aufgebracht ist,
wobei der Wirkstoff Fentanyl oder ein Analogstoff des Fentanyls ausgewählt aus Alfentanil, Carfentanil, Lofentanil, Remifentanil und Trefentanil oder ein Salz eines dieser Wirkstoffe ist und
wobei die Matrixschicht frei von Gelbildnern ist und als haftklebendes Polymer ein Gemisch aus einem Polyisobutylen A und einem Polyisobutylen B enthält,
wobei der Gehalt des Polyisobutylens A zum Polyisobutylen B in der Matrixschicht im Bereich von 20% (A) : 80% (B) bis 40% (A) : 60% (B) liegt, jeweils bezogen auf das Gesamtgewicht der Polyisobutylene A und B, und
wobei das Polyisobutylen A ein Speichermodul G' aufweist, dessen Wert im Temperaturbereich von 10°C bis 40°C im Wesentlichen konstant ist und
das Polyisobutylen B ein Speichermodul G' aufweist, dessen Wert im Temperaturbereich von 10°C bis 40°C mit steigender Temperatur kontinuierlich abnimmt,
wobei das Speichermodul G' im linearen viskoelastischen Bereich bei einer Frequenz von 10 rad/sec unter Verwendung eines Rheometers mit paralleler Plattengeometrie und parallelen Platten gemessen wird, und
wobei die haftklebende Matrixschicht ungelösten Wirkstoff in Form von Wirkstoffpartikeln enthält, und nach Applikation auf der Haut für die Dauer der vorgesehenen Tragezeit, einen Restwirkstoffgehalt von unter 35%, vorzugsweise unter 25% des Ausgangsgehalts an Wirkstoff aufweist.

2. Transdermales therapeutisches System zur Verwendung zur Linderung von Schmerz gemäß Anspruch 1, wobei die haftklebende Matrixschicht ungelösten Wirkstoff in Form von Wirkstoffpartikeln enthält, wobei in der Matrixschicht der Wirkstoff in einer Menge von 3 bis 10 Gew.-%, das Polyisobutylen A in einer Menge von 10 bis 25 Gew.-% und das Polyisobutylen B in einer Menge von 30 bis 50 Gew.-%, jeweils bezogen auf das Gesamtgewicht der Matrixschicht, enthalten ist.

3. Transdermales therapeutisches System zur Verwendung zur Linderung von Schmerz nach einem der Ansprüche 1 oder 2, wobei für das Polyisobutylen A im Temperaturbereich von 10°C bis 40°C alle Werte des Speichermoduls G' von dem Wert für das Speichermodul G' bei 40°C um nicht mehr als 50%, bevorzugt um nicht mehr als 25% abweichen.

4. Transdermales therapeutisches System zur Verwendung zur Linderung von Schmerz nach einem der vorstehenden Ansprüche, wobei für das Polyisobutylen B das Speichermodul G' bei 10°C mindestens das 2-fache, bevorzugt mindestens das 3-fache des Speichermoduls G' bei 80°C beträgt.

5. Transdermales therapeutisches System zur Verwendung zur Linderung von Schmerz nach einem der vorstehenden Ansprüche, wobei der Gehalt des Polyisobutylens A zum Polyisobutylen B in der Matrixschicht im Bereich von 25% (A) : 75% (B) bis 35% (A) : 65% (B) liegt, jeweils bezogen auf das Gesamtgewicht der Polyisobutylene A und B.

6. Transdermales therapeutisches System zur Verwendung zur Linderung von Schmerz nach einem der vorstehenden Ansprüche, wobei es sich bei dem Polyisobutylen A und bei dem Polyisobutylen B jeweils um einzelne Polyisobutylene mit unterschiedlichen mittleren Molekulargewichten handelt.

7. Transdermales therapeutisches System zur Verwendung zur Linderung von Schmerz nach einem der Ansprüche 1-7, wobei die Matrixschicht einen Permeationsverstärker enthält, bei dem es sich bevorzugt um Isopropylmyristat oder Oleyloleat handelt und/oder wobei die Matrixschicht einen Klebrigmacher (tackifier) enthält, bei dem es sich bevorzugt um ein Polybuten oder einen hydrierten oder nicht hydrierten Kolophoniumester handelt.

8. Transdermales therapeutisches System zur Verwendung zur Linderung von Schmerz nach einem der vorstehenden Ansprüche, **dadurch gekennzeichnet, dass** die Wirkstoffmenge für eine Applikationsdauer von 3 Tagen ausreichend ist und der Wirkstoff in der Matrixschicht in einer Konzentration im Bereich von 4-6 Gew.-% und bevorzugt im Bereich von 5-6 Gew.-% vorhanden ist (bezogen auf das Gewicht der Matrixschicht).

9. Transdermales therapeutisches System zur Verwendung zur Linderung von Schmerz nach einem der vorstehenden Ansprüche, **dadurch gekennzeichnet, dass** in der haftklebenden Matrixschicht neben dem Wirkstoff, dem Polyisobutylen A und dem Polyisobutylen B nur noch ein Klebrigmacher, bevorzugt ein Polybuten oder ein hydrierter Kolophoniumester und ein Permeationsverstärker, bevorzugt Isopropylmyristat oder Oleyloleat vorhanden ist, wobei bevorzugt der Klebrigmacher in einer Menge im Bereich von 23 bis 28% und der Permeationsverstärker in einer Menge im Bereich von 8 bis 15 % vorhanden sind, jeweils bezogen auf das Gesamtgewicht der Matrixschicht.

10. Verfahren zur Herstellung eines transdermalen therapeutischen Systems nach einem der Ansprüche 1-9, bei dem die haftklebende Matrixschicht frei von Gelbildnern ist und einen Permeationsverstärker enthält, wobei der Wirkstoff im Permeationsverstärker dispergiert wird,
das Polyisobutylen A und das Polyisobutylen B, die wie in Ansprüchen 1 bis 7 definiert sind, jeweils in einem geeigneten Lösungsmittel verteilt werden, anschließend die beiden polymerhaltigen Lösungen homogen gemischt werden,
die polymerhaltigen Lösungen mit dem dispergierten Wirkstoff und gegebenenfalls weitere Bestandteile gemischt werden, bis eine einheitliche Masse entsteht,
die so entstandene Masse auf die Abziehschicht oder die Rückschicht aufgebracht wird,
das Lösungsmittel entfernt wird,
die Rückschicht bzw. die Abziehschicht auflaminiert wird und
transdermale therapeutische Systeme der gewünschten Größe ausgeschnitten oder ausgestanzt werden.

11. Transdermales therapeutisches System zur Verwendung zur Linderung von Schmerz nach einem der Ansprüche 1-9, bei dem die haftklebende Matrixschicht einen Permeationsverstärker enthält, erhältlich nach einem Verfahren nach Anspruch 10.

12. Verwendung einer haftklebenden Matrixschicht, wobei die Matrixschicht frei von Gelbildnern ist und als haftklebendes Polymer ein Gemisch aus einem Polyisobutylen A und einem Polyisobutylen B enthält,
wobei der Gehalt des Polyisobutylens A zum Polyisobutylen B in der Matrixschicht im Bereich von 20% (A) : 80% (B) bis 40% (A) : 60% (B) liegt, jeweils bezogen auf das Gesamtgewicht der Polyisobutylene A und B, und
wobei das Polyisobutylen A ein Speichermodul G' aufweist, dessen Wert im Temperaturbereich von 10°C bis 40°C im Wesentlichen konstant ist und
das Polyisobutylen B ein Speichermodul G' aufweist, dessen Wert im Temperaturbereich von 10°C bis 40°C mit steigender Temperatur kontinuierlich abnimmt,
wobei das Speichermodul G' im linearen viskoelastischen Bereich bei einer Frequenz von 10 rad/sec unter Verwendung eines Rheometers mit paralleler Plattengeometrie und parallelen Platten gemessen wird, und
wobei die haftklebende Matrixschicht ungelösten Wirkstoff in Form von Wirkstoffpartikeln enthält, zur Herstellung eines transdermalen therapeutischen Systems, bestehend aus
a) einer Rückschicht,
b) einer haftklebenden Matrixschicht, die den Wirkstoff enthält,
und
c) einer Abziehschicht (Release-Liner),
sowie gegebenenfalls
b1) einer zusätzlichen Klebstoffschicht oder einem Overtape, die die Haftung des transdermalen therapeutischen Systems auf der Haut verbessern und die auf der haftklebenden Matrixschicht aufgebracht sind, oder
b2) einer Membran, die die Freisetzung des Wirkstoffes kontrolliert und auf der sich eine Klebstoffschicht befindet, wobei die Membran auf der haftklebenden Matrixschicht aufgebracht ist,
wobei der Wirkstoff Fentanyl oder ein Analogstoff des Fentanyls ausgewählt aus Alfentanil, Carfentanil, Lofentanil, Remifentanil und Trefentanil oder ein Salz eines dieser Wirkstoffe ist und das gegen Missbrauch oder Fehlgebrauch geschützt ist.

13. Verwendung einer haftklebenden Matrixschicht gemäß Anspruch 12 zur Herstellung eines transdermalen therapeutischen Systems, das Schmerz während einer vorgesehenen Tragezeit von 3 bis 7 Tagen lindert,
wobei die Matrixschicht des herzustellenden transdermalen therapeutischen Systems nach Applikation auf der Haut für die vorgesehene Tragezeit einen Restwirkstoffgehalt von unter 35%, vorzugsweise unter 25% des Ausgangsgehalts an Wirkstoff aufweist.

14. Verwendung einer haftklebenden Matrixschicht gemäß Anspruch 13, wobei die Matrixschicht frei von Gelbildnern ist und als haftklebendes Polymer ein Gemisch aus einem Polyisobutylen A und einem Polyisobutylen B enthält, wobei der Gehalt des Polyisobutylens A zum Polyisobutylen B in der Matrixschicht im Bereich von 20% (A) : 80% (B) bis 40% (A) : 60% (B) liegt, jeweils bezogen auf das Gesamtgewicht der Polyisobutylene A und B,
wobei das Polyisobutylen A ein Speichermodul G' aufweist, dessen Wert im Temperaturbereich von 10°C bis 40°C im Wesentlichen konstant ist und
das Polyisobutylen B ein Speichermodul G' aufweist, dessen Wert im Temperaturbereich von 10°C bis 40°C mit steigender Temperatur kontinuierlich abnimmt,
wobei das Speichermodul G' im linearen viskoelastischen Bereich bei einer Frequenz von 10 rad/sec unter Verwendung eines Rheometers mit paralleler Plattengeometrie und parallelen Platten gemessen wird, und
wobei die haftklebende Matrixschicht ungelösten Wirkstoff in Form von Wirkstoffpartikeln enthält, zur Bereitstellung eines transdermalen therapeutischen Systems, das eine Abgaberate von mehr als 100 µg/h, vorzugsweise mindestens 200 µg/h aufweist, wobei das transdermale therapeutische System bevorzugt eine Größe von weniger als 50 cm², vorzugsweise von weniger als 45 cm² aufweist.

## Claims

1. A transdermal therapeutic system for use in the relief of pain by administering an active ingredient through the skin during an intended time of wearing of from 3 to 7 days consisting of
a) a back layer,
b) a pressure-sensitive matrix layer containing the active ingredient, and
c) a release-liner,
as well as optionally
b1) an additional adhesive layer or an overtape which improve the adhesion of the transdermal therapeutic system to the skin and which are applied to the pressure-sensitive matrix layer, or
b2) a membrane which controls the release of the active ingredient and on which an adhesive layer is located wherein the membrane is applied to the pressure-sensitive matrix layer,
wherein the active ingredient is fentanyl or an analogue of the fentanyl selected from alfentanil, carfentanil, lofentanil, remifentanil, and trefentanil or a salt of one of said active ingredients and
wherein the matrix layer is free from gel-forming agents and contains a mixture of a polyisobutylene A and a polyisobutylene B as a pressure-sensitive polymer,
wherein the content of the polyisobutylene A to the polyisobutylene B in the matrix layer is in the range of from 20% (A) : 80% (B) to 40% (A) : 60% (B) each based on the total weight of the polyisobutylenes A and B, and
wherein the polyisobutylene A has a storage modulus G' the value of which is substantially constant the temperature range of from 10°C to 40°C, and
the polyisobutylene B has a storage modulus G' the value of which continuously decreases with an increasing temperature in the temperature range of from 10°C to 40°C,
wherein the storage modulus G' is measured in the linear viscoelastic range at a frequency of 10 rad/sec using a rheometer with a parallel plate geometry and parallel plates, and
wherein the pressure-sensitive matrix layer contains undissolved active ingredient in the form of active ingredient particles and after application to the skin for the duration of the intended time of wearing has a residual active ingredient content of less than 35%, preferably less than 25% of the initial content of active ingredient.

2. The transdermal therapeutic system for use in the relief of pain according to claim 1, wherein the pressure-sensitive matrix layer contains undissolved active ingredient in the form of active ingredient particles, wherein in the matrix layer the active ingredient is contained in an amount of from 3 to 10% wt., the polyisobutylene A in an amount of from 10 to 25% wt., and the polyisobutylene B in an amount of from 30 to 50% wt., each based on the total weight of the matrix layer.

3. The transdermal therapeutic system for use in the relief of pain according to any one of claims 1 or 2, wherein in case of polyisobutylene A in the temperature range of from 10°C to 40°C all values of the storage modulus G' do not deviate from the value for the storage modulus G' at 40°C by more than 50%, preferably by more than 25%.

4. The transdermal therapeutic system for use in the relief of pain according to any of the preceding claims, wherein in case of polyisobutylene B the storage modulus G' at 10°C is at least 2 times, preferably at least 3 times the storage modulus G' at 80°C.

5. The transdermal therapeutic system for use in the relief of pain according to any of the preceding claims, wherein the content of the polyisobutylene A to the polyisobutylene B in the matrix layer is in the range of from 25% (A) : 75% (B) to 35% (A) : 65% (B), each based on the total weight of the polyisobutylenes A and B.

6. The transdermal therapeutic system for use in the relief of pain according to any of the preceding claims, wherein the polyisobutylene A and the polyisobutylene B each are individual polyisobutylenes with different mean molecular weights.

7. The transdermal therapeutic system for use in the relief of pain according to any one of claims 1-7, wherein the matrix layer contains a permeation enhancer which preferably is isopropylmyristate or oleyloleate and/or wherein the matrix layer contains a tackifier which preferably is a polybutene or a hydrogenated or non-hydrogenated rosin ester.

8. The transdermal therapeutic system for use in the relief of pain according to any of the preceding claims, **characterized in that** the amount of active ingredient is sufficient for a period of application of 3 days and the active ingredient is present in the matrix layer in a concentration in the range of from 4-6% wt., and preferably in the range of from 5-6% wt. (based on the weight of the matrix layer).

9. The transdermal therapeutic system for use in the relief of pain according to any of the preceding claims, **characterized in that** in the pressure-sensitive matrix layer in addition to the active ingredient, the polyisobutylene A and the polyisobutylene B there is only present a tackifier, preferably a polybutene or a hydrogenated rosin ester, and a permeation enhancer, preferably isopropylmyristate or oleyloleate, preferably wherein the tackifier is present in an amount in the range of from 23 to 28%, and the permeation enhancer is present in an amount in the range of from 8 to 15%, each based on the total weight of the matrix layer.

10. A method for the preparation of a transdermal therapeutic system according to any one of claims 1-9, in which the pressure-sensitive matrix layer is free from gel-forming agents and contains a permeation enhancer, wherein the active ingredient is dispersed in the permeation enhancer,
the polyisobutylene A and the polyisobutylene B as defined in claims 1 to 7 each are dispersed in a suitable solvent, subsequently both polymer-containing solutions are homogenously mixed,
the polymer-containing solutions are mixed with the dispersed active ingredient and optionally further components until a uniform mass is formed,
the thus formed mass is applied to the stripping layer or the back layer,
the solvent is removed,
the back layer or the stripping layer, respectively is laminated, and
transdermal therapeutic systems of the desired size are cut or die-cut.

11. The transdermal therapeutic system for use in the relief of pain according to any one of claims 1-9, in which the pressure-sensitive matrix layer contains a permeation enhancer which can be obtained according to a method of claim 10.

12. Use of a pressure-sensitive matrix layer, wherein the matrix layer is free from gel-forming agents and contains a mixture of a polyisobutylene A and a polyisobutylene B as a pressure-sensitive polymer,
wherein the content of the polyisobutylene A to the polyisobutylene B in the matrix layer is in the range of from 20% (A) : 80% (B) to 40% (A) : 60% (B), each based on the total weight of the polyisobutylenes A and B, and
wherein the polyisobutylene A has a storage modulus G' the value of which is substantially constant the temperature range of from 10°C to 40°C, and
the polyisobutylene B has a storage modulus G' the value of which continuously decreases with an increasing temperature in the temperature range of from 10°C to 40°C,
wherein the storage modulus G' is measured in the linear viscoelastic range at a frequency of 10 rad/sec using a rheometer with a parallel plate geometry and parallel plates, and
wherein the pressure-sensitive matrix layer contains undissolved active ingredient in the form of active ingredient particles, for the preparation of a transdermal therapeutic system consisting of
a) a back layer,
b) a pressure-sensitive matrix layer containing the active ingredient, and
c) a release-liner,
as well as optionally
b1) an additional adhesive layer or an overtape which improve the adhesion of the transdermal therapeutic system to the skin and which are applied to the pressure-sensitive matrix layer, or
b2) a membrane which controls the release of the active ingredient and on which an adhesive layer is located wherein the membrane is applied to the pressure-sensitive matrix layer,
wherein the active ingredient is fentanyl or an analogue of the fentanyl selected from alfentanil, carfentanil, lofentanil, remifentanil, and trefentanil or a salt of one of said active ingredients and which is protected against abuse or misuse.

13. Use of a pressure-sensitive matrix layer according to claim 12 for the preparation of a transdermal therapeutic system relieving pain during an intended time of wearing of from 3 to 7 days,
wherein the matrix layer of the transdermal therapeutic system to be prepared after application to the skin for the intended time of wearing has a residual active ingredient content of less than 35%, preferably less than 25% of the initial content of active ingredient.

14. Use of a pressure-sensitive matrix layer according to claim 13, wherein the matrix layer is free from gel-forming agents and contains a mixture of a polyisobutylene A and a polyisobutylene B as a pressure-sensitive polymer, wherein the content of the polyisobutylene A to the polyisobutylene B in the matrix layer is in the range of from 20% (A) : 80% (B) to 40% (A) : 60% (B), each based on the total weight of the polyisobutylenes A and B,
wherein the polyisobutylene A has a storage modulus G' the value of which is substantially constant the temperature range of from 10°C to 40°C, and
the polyisobutylene B has a storage modulus G' the value of which continuously decreases with an increasing temperature in the temperature range of from 10°C to 40°C,
wherein the storage modulus G' is measured in the linear viscoelastic range at a frequency of 10 rad/sec using a rheometer with a parallel plate geometry and parallel plates, and
wherein the pressure-sensitive matrix layer contains undissolved active ingredient in the form of active ingredient particles, for providing a transdermal therapeutic system which has a delivery rate greater than 100 µg/h, preferably at least 200 µg/h, preferably wherein the transdermal therapeutic system has a size of less than 50 cm², preferably of less than 45 cm².

## Revendications

1. Système thérapeutique transdermique pour l'utilisation dans le soulagement de la douleur par administration d'un principe actif à travers la peau pendant une durée de port prévue de 3 à 7 jours, consistant de :
a) une couche de support,
b) une couche de matrice adhésive contenant le principe actif,
et
c) une couche de protection (Release-Liner),
ainsi que, le cas échéant,
b1) une couche adhésive supplémentaire ou un surruban, qui améliorent l'adhérence du système thérapeutique transdermique sur la peau et qui sont appliqués sur la couche de matrice adhésive, ou
b2) une membrane qui contrôle la libération du principe actif et sur laquelle se trouve une couche adhésive, la membrane étant appliquée sur la couche de matrice adhésive,
le principe actif étant le fentanyl ou un analogue du fentanyl choisi parmi l'alfentanil, le carfentanil, le lofentanil, le remifentanil et le trefentanil, ou un sel de ces principes actifs, et
la couche de matrice étant exempte de gélifiants et contenant un mélange de polyisobutylène A et de polyisobutylène B comme polymère adhésif,
la teneur en polyisobutylène A par rapport au polyisobutylène B dans la couche de matrice étant comprise dans la plage de 20% (A) : 80% (B) à 40% (A) : 60% (B), par rapport au poids total des polyisobutylènes A et B, et
le polyisobutylène A ayant un module de stockage G' dont la valeur est essentiellement constante dans la plage de température de 10°C à 40°C, et
le polyisobutylène B ayant un module de stockage G' dont la valeur diminue continuellement avec l'augmentation de la température dans la plage de température de 10°C à 40°C,
le module de stockage G' étant mesuré dans la plage viscoélastique linéaire à une fréquence de 10 rad/sec en utilisant un rhéomètre avec une géométrie de plaques parallèles et des plaques parallèles, et
la couche de matrice adhésive contenant le principe actif non dissous sous forme de particules de principe actif, et après application sur la peau pendant la durée de port prévue, ayant une teneur en principe actif résiduel inférieure à 35%, de préférence inférieure à 25% de la teneur initiale en principe actif.

2. Système thérapeutique transdermique pour l'utilisation dans le soulagement de la douleur selon la revendication 1, la couche de matrice adhésive contenant le principe actif non dissous sous forme de particules de principe actif, la couche de matrice contenant le principe actif en une quantité de 3 à 10 % en poids, le polyisobutylène A en une quantité de 10 à 25 % en poids et le polyisobutylène B en une quantité de 30 à 50 % en poids, par rapport au poids total de la couche de matrice.

3. Système thérapeutique transdermique pour l'utilisation dans le soulagement de la douleur selon l'une des revendications 1 ou 2, dans lequel, pour le polyisobutylène A, tous les valeurs du module de stockage G' dans la plage de température de 10°C à 40°C ne s'écartent pas de plus de 50%, de préférence de pas plus de 25%, de la valeur du module de stockage G' à 40°C.

4. Système thérapeutique transdermique pour l'utilisation dans le soulagement de la douleur selon l'une des revendications précédentes, le module de stockage G' du polyisobutylène B à 10°C étant au moins deux fois, de préférence au moins trois fois, le module de stockage G' à 80°C.

5. Système thérapeutique transdermique pour l'utilisation dans le soulagement de la douleur selon l'une des revendications précédentes, la teneur en polyisobutylène A par rapport au polyisobutylène B dans la couche de matrice étant comprise dans la plage de 25% (A) : 75% (B) à 35% (A) : 65% (B), par rapport au poids total des polyisobutylènes A et B.

6. Système thérapeutique transdermique pour l'utilisation dans le soulagement de la douleur selon l'une des revendications précédentes, le polyisobutylène A et le polyisobutylène B étant chacun des polyisobutylènes individuels avec des poids moléculaires moyens différents.

7. Système thérapeutique transdermique pour l'utilisation dans le soulagement de la douleur selon l'une des revendications 1 à 7, la couche de matrice contenant un promoteur de perméation, de préférence l'isopropylmyristate ou l'oléyloléate, et/ou la couche de matrice contenant un agent collant (tackifier), de préférence un polybutène ou un ester de colophane hydrogéné ou non hydrogéné.

8. Système thérapeutique transdermique pour l'utilisation dans le soulagement de la douleur selon l'une des revendications précédentes, **caractérisé en ce que** la quantité de principe actif est suffisante pour une durée d'application de 3 jours et le principe actif étant présent dans la couche de matrice à une concentration de 4 à 6 % en poids, de préférence de 5 à 6 % en poids (par rapport au poids de la couche de matrice).

9. Système thérapeutique transdermique pour l'utilisation dans le soulagement de la douleur selon l'une des revendications précédentes, **caractérisé en ce que**, dans la couche de matrice adhésive, outre le principe actif, le polyisobutylène A et le polyisobutylène B, seul un agent collant, de préférence un polybutène ou un ester de colophane hydrogéné, et un promoteur de perméation, de préférence l'isopropylmyristate ou l'oléyloléate, sont présents, de préférence l'agent collant étant présent en une quantité de 23 à 28% et le promoteur de perméation en une quantité de 8 à 15%, par rapport au poids total de la couche de matrice.

10. Procédé de fabrication d'un système thérapeutique transdermique selon l'une des revendications 1 à 9, la couche de matrice adhésive étant exempte de gélifiants et contenant un promoteur de perméation, le principe actif étant dispersé dans le promoteur de perméation,
le polyisobutylène A et le polyisobutylène B, qui sont tels que définis dans les revendications 1 à 7, étant chacun dispersés dans un solvant approprié, les deux solutions polymères étant ensuite mélangées de manière homogène,
les solutions polymères étant mélangées avec le principe actif dispersé et éventuellement d'autres composants jusqu'à obtention d'une masse homogène,
la masse ainsi obtenue étant appliquée sur la couche de protection ou la couche de support,
le solvant étant éliminé,
la couche de support ou la couche de protection étant laminée sur celle-ci et
des systèmes thérapeutiques transdermiques de la taille souhaitée étant découpés ou poinçonnés.

11. Système thérapeutique transdermique pour l'utilisation dans le soulagement de la douleur selon l'une des revendications 1 à 9, la couche de matrice adhésive contenant un promoteur de perméation, obtenu selon un procédé selon la revendication 10.

12. Utilisation d'une couche de matrice adhésive, la couche de matrice étant exempte de gélifiants et contenant un mélange de polyisobutylène A et de polyisobutylène B comme polymère adhésif,
dans lequel la teneur en polyisobutylène A par rapport au polyisobutylène B dans la couche de matrice est comprise dans la plage de 20 % (A) : 80 % (B) à 40 % (A) : 60 % (B), par rapport au poids total des polyisobutylènes A et B, et
dans lequel le polyisobutylène A a un module de stockage G' dont la valeur est essentiellement constante dans la plage de température de 10°C à 40°C et
le polyisobutylène B a un module de stockage G' dont la valeur diminue continuellement avec l'augmentation de la température dans la plage de température de 10°C à 40°C,
dans lequel le module de stockage G' est mesuré dans la plage viscoélastique linéaire à une fréquence de 10 rad/sec en utilisant un rhéomètre avec une géométrie de plaques parallèles et des plaques parallèles, et
dans lequel la couche de matrice adhésive contient le principe actif non dissous sous forme de particules de principe actif, pour la fabrication d'un système thérapeutique transdermique, consistant de
a) une couche de support,
b) une couche de matrice adhésive contenant le principe actif,
et
c) une couche de protection (Release-Liner),
ainsi que, le cas échéant,
b1) une couche adhésive supplémentaire ou un surruban, qui améliorent l'adhérence du système thérapeutique transdermique sur la peau et qui sont appliqués sur la couche de matrice adhésive, ou
b2) une membrane qui contrôle la libération du principe actif et sur laquelle se trouve une couche adhésive, la membrane étant appliquée sur la couche de matrice adhésive,
dans lequel le principe actif est le fentanyl ou un analogue du fentanyl choisi parmi l'alfentanil, le carfentanil, le lofentanil, le remifentanil et le trefentanil ou un sel de l'un de ces principes actifs et est protégé contre l'abus ou le mésusage.

13. Utilisation d'une couche de matrice adhésive selon la revendication 12 pour la fabrication d'un système thérapeutique transdermique qui soulage la douleur pendant une durée de port prévue de 3 à 7 jours,
dans lequel la couche de matrice du système thérapeutique transdermique fabriqué présente, après application sur la peau pendant la durée de port prévue, une teneur résiduelle en principe actif inférieure à 35 %, de préférence inférieure à 25 % de la teneur initiale en principe actif.

14. Utilisation d'une couche de matrice adhésive selon la revendication 13, dans lequel la couche de matrice est exempte de gélifiants et contient un mélange de polyisobutylène A et de polyisobutylène B comme polymère adhésif, dans lequel la teneur en polyisobutylène A par rapport au polyisobutylène B dans la couche de matrice est comprise dans la plage de 20 % (A) : 80 % (B) à 40 % (A) : 60 % (B), par rapport au poids total des polyisobutylènes A et B,
dans lequel le polyisobutylène A a un module de stockage G' dont la valeur est essentiellement constante dans la plage de température de 10°C à 40°C et
le polyisobutylène B a un module de stockage G' dont la valeur diminue continuellement avec l'augmentation de la température dans la plage de température de 10°C à 40°C,
dans lequel le module de stockage G' est mesuré dans la plage viscoélastique linéaire à une fréquence de 10 rad/sec en utilisant un rhéomètre avec une géométrie de plaques parallèles et des plaques parallèles, et
dans lequel la couche de matrice adhésive contient le principe actif non dissous sous forme de particules de principe actif, pour fournir un système thérapeutique transdermique ayant un taux de libération supérieur à 100 µg/h, de préférence au moins 200 µg/h, le système thérapeutique transdermique ayant de préférence une taille inférieure à 50 cm², de préférence inférieure à 45 cm².
